# EUROPEAN PATENT SPECIFICATION

(11) **EP 0 986 387 B1**
(45) Date of publication and mention of the grant of the patent: **02.04.2003**
(21) Application number: 98921688.2
(22) Date of filing: 05.06.1998
(51) Int. Cl.: A61K 31/435, A61K 31/22

(54) **FARNESYL TRANSFERASE INHIBITORS IN COMBINATION WITH HMG CoA REDUCTASE INHIBITORS FOR THE TREATMENT OF CANCER**
FARNESYL TRANSFERASEINHIBITOREN IN KOMBINATION MIT HMG CoA REDUKTASEINHIBITOR UND DEREN VERWENDUNG IN DER KREBSTHERAPIE
INHIBITEURS DE FARNESYLE TRANSFERASE ASSOCIES A DES INHIBITEURS DE HMG COA REDUCTASE, ET LEUR UTILISATION ANTICANCEREUSE

(30) Priority: 16.06.1997 US 49638 P
(43) Date of publication of application: 22.03.2000
(73) Proprietor: Pfizer Products Inc., Groton, Connecticut 06340 (US)
(72) Inventor: KAJIJI, Shama, Mohammed, Mystic, CT 06355 (US)
(74) Representative: Ruddock, Keith Stephen
(86) International application number: IB9800881
(87) International publication number: WO98057633

(56) References cited:
- WO-A-97/05902

## Description

This invention relates to the use of a farnesyl transferase (FTase) inhibitor in combination with a hydroxymethylglutaryl coenzyme A (HMG CoA) reductase inhibitor for the preparation of a medicament for treating cancer in a mammal.

Oncogenes are genes that, when activated, encode protein components of signal transduction pathways which lead to the abnormal stimulation of cell growth and mitogenesis. Oncogene expression in cultured cells leads to cellular transformation, characterized by the ability of cells to grow in soft agar and the growth of cells as dense foci lacking the contact inhibition exhibited by non-transformed cells.

Mutation and/or overexpression of certain oncogenes is frequently associated with human cancers and other disorders involving abnormal (i.e., unregulated) cell growth. For example, the growth of benign and malignant tumors can be caused by the expression of an activated *Ras* oncogene or by activation of the *Ras* protein by another gene that has undergone oncogenic mutation. The abnormal growth of cells that occurs in the benign and malignant cells of other proliferative disorders can be caused by aberrant *Ras* activation. Mutated, oncogenic forms of *Ras* are frequently found in many human cancers, most notably in more than 50% of colon and pancreatic carcinomas (Kohl et al., Science, Vol. 260, 1834 to 1837, 1993). The *Ras* oncogene is expressed in about 40% of solid malignant tumors that are unresponsive to conventional chemotherapies. The K-*Ras* isoform is expressed in about 90% of pancreatic tumors and about 40% of colorectal and lung cancers. The H-Ras isoform is expressed in about 40% of head and neck cancers. The N-Ras isoform is expressed in most thyroid cancers and about 25% of acute myeloid leukemias. To acquire the potential to transform normal cells into cancer cells or benign cells that exhibit abnormal growth, as defined below, the precursor of the *Ras* oncoprotein must undergo farnesylation of the cysteine residue located in a carboxyl-terminal tetrapeptide. Inhibitors of the enzyme that catalyzes this modification, famesyl protein transferase, are therefore useful as anticancer agents for tumors in which *Ras* contributes to transformation.

The K-*Ras* isoform can be both farnesylated and geranyl-geranylated in intact cells. Potent inhibitors of the enzyme farnesyl (FTase) that are highly selective for FTase versus geranylgeranyl transferase I (GGTase I) can be incapable of blocking prenylation of mutant K-*Ras* and therefore ineffective at inhibiting growth of K-*Ras* expressing tumor cells.

The present inventor has found that the administration of a low dose HMG CoA reductase inhibitor in combination with a potent selective FTase inhibitor will block K-*Ras* prenylation and K-*Ras* function, as well as H-*Ras* prenylation and function. The activity of the protein prenyl transferases FTase and GGTase I is dependent on the concentrations of the isoprenoid substrates, farnesyl- and geranylgeranyl-pyrophosphates, respectively. Mevalonate is the first committed intermediate in the isoprenoid pathway, and its synthesis is dependent on the activity of HMG CoA reductase. Compounds such as lovastatin and compactin, which are tight binding inhibitors of HMG CoA reductase, block mevalonate formation and thus block the isoprenoid pathway. They therefore inhibit both FTase and GGTase I.

The therapeutic effect of compounds from the two above classes of drugs (FTase inhibitor and HMG CoA reductase inhibitor) is believed to be synergistic. The present inventor has found that the combined administration of an FTase inhibitor and an HMG CoA reductase inhibitor overcomes the limitations of each given separately. The combination is therefore expected to be effective in cases where either agent alone would not be effective.

Japanese Patent Application JP7316076A, which was published on December 5, 1995, refers to an anticancer pharmaceutical composition that contains limonene, which, while not a FTase inhibitor, has been shown to impair the incorporation of mevalonic acid-derived isoprene compounds into *Ras* and *Ras* related proteins, and pravastatin, which is an HMG CoA reductase inhibitor.

The present invention relates to a pharmaceutical composition for the treatment of cancer or a benign proliferative disorder in a mammal, including a human, comprising a FTase inhibitor, an HMG CoA reductase inhibitor and a pharmaceutically acceptable carrier, wherein the active ingredients in such composition (i.e., the FTase inhibitor and the HMG CoA reductase inhibitor) are present in amounts that render the composition effective in the treatment of cancer or a benign proliferative disorder.

This invention also can be used for a method of treating cancer or a benign proliferative disorder in a mammal, including a human, said method comprising administering to said mammal an anticancer or antiproliferative effective amount of a pharmaceutical composition comprising a FTase inhibitor, an HMG CoA reductase inhibitor and a pharmaceutically acceptable carrier.

This invention also can be used for a method of treating cancer or a benign proliferative disorder in a mammal, including a human, said method comprising administering to said mammal a FTase inhibitor and an HMG CoA reductase inhibitor in amounts that render the combination of such two active agents effective in the treatment of cancer or a benign proliferative disorder.

This invention also relates to a pharmaceutical composition for inhibiting the abnormal growth of cells in a mammal, including a human, comprising a FTase inhibitor, an HMG CoA reductase inhibitor and a pharmaceutically acceptable carrier, wherein the active ingredients in such composition (i.e., the FTase inhibitor and the HMG CoA reductase inhibitor) are present in amounts that render the composition effective in inhibiting the abnormal growth of cells.

This invention also can be used for a method of inhibiting the abnormal growth of cells in a mammal, including a human, said method comprising administering to said mammal a FTase inhibitor and an HMG CoA reductase inhibitor in amounts that render the combination of such two active ingredients effective in inhibiting the abnormal growth of cells.

The term "treating, as used herein, refers to preventing, or retarding or inhibiting the progress of the disorder to which such term is applied.

"Abnormal cell growth", as used herein, refers to cell growth that is independent of normal regulatory mechanisms (e.g., loss of contact inhibition). This includes the abnormal growth of: (1) tumor cells (tumors) expressing an activated *Ras* oncogene; (2) tumor cells in which the *Ras* protein is activated as a result of oncogenic mutation in another gene; and (3) benign and malignant cells of other proliferative diseases in which aberrant *Ras* activation occurs.

Examples of such benign proliferative diseases are psoriasis, benign prostatic hypertrophy and restenosis.

Patients that can be treated with a FTase inhibitor in combination with an HMG CoA reductase inhibitor by using the pharmaceutical compositions of the invention include, for example, patients that have been diagnosed as having lung cancer, bone cancer, pancreatic cancer, skin cancer, cancer of the head and neck, cutaneous or intraocular melanoma, uterine cancer, ovarian cancer, rectal cancer, cancer of the anal region, stomach cancer, colon cancer, breast cancer, gynecologic tumors (e.g., uterine sarcomas, carcinoma of the fallopian tubes, carcinoma of the endometrium, carcinoma of the cervix, carcinoma of the vagina or carcinoma of the vulva), Hodgkin's disease, cancer of the esophagus, cancer of the small intestine, cancer of the endocrine system (e.g., cancer of the thyroid, parathyroid or adrenal glands), sarcomas of soft tissues, cancer of the urethra, cancer of the penis, prostate cancer, chronic or acute leukemia, solid tumors of childhood, lymphocytic lymphonas, cancer of the bladder, cancer of the kidney or ureter (e.g., renal cell carcinoma, carcinoma of the renal pelvis), or neoplasms of the central nervous system (e.g., primary CNS lymphona, spinal axis tumors, brain stem gliomas or pituitary adenomas).

Patients that can be treated with a FTase inhibitor in combination with an HMG CoA reduction inhibitor by using the pharmaceutical compositions of the invention also include patients suffering from abnormal cell growth, as defined above.

More specific embodiments of this invention relate to the above pharmaceutical compositions and their use in the preparation of a medicament for methods of treatment wherein the FTase inhibitor is selected from:
(a) compounds of the formula 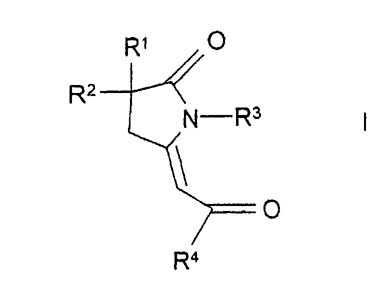 wherein R¹ and R² are independently selected from the group consisting of -(CH₂)ₚ(5-10 membered heterocycles), -(CH₂)ₚ(C₆-C₁₀ aryl), allyl, propargyl and C₁-C₆ alkyl wherein p is 0 to 3, said alkyl and the alkyl moieties of said R¹ and R² groups are optionally substituted by 1 to 3 R⁹ substituents, and the aryl and heterocyclic moieties of said R¹ and R² groups are optionally substituted by 1 to 3 substituents independently selected from halo and R⁹;
   R³ is -(CH₂)ₘ(1- or 2-adamantyl), -(CH₂)ₘ(C₃-C₁₀ cycloalkyl), -(CH₂)ₘ(C₆-C₁₀ aryl), C₁-C₁₀ alkyl, 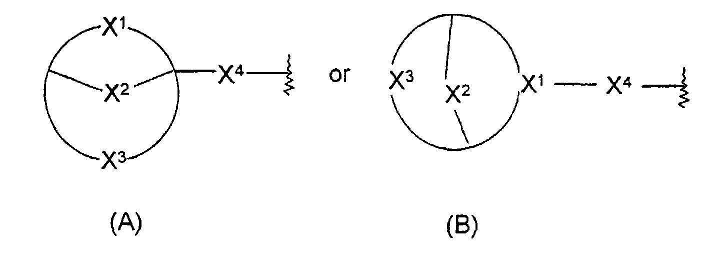 wherein m is 0 to 6, and said cycloalkyl and alkyl optionally contain 1 or 2 double or triple bonds;
   X¹, X², and X³ are each independently C₁-C₇ alkylene optionally containing 1 or 2 double or triple bonds, X⁴ is a bond or C₁-C₇ alkylene optionally containing 1 or 2 double or triple bonds, and, in formula (B), the X⁴ moiety is attached to the X¹ moiety at any available carbon in the X¹ moiety;
   R⁴ is C₆-C₁₀ aryl, 5-10 membered heterocyclyl or C₁-C₆ alkyl wherein each of said R⁴ groups is optionally substituted by 1 to 3 R⁵ substituents;
   each R⁵ is independently selected from the group consisting of halo, nitro, cyano, phenyl, -C(O)OR⁶, -SO₂NR⁶R⁷, -NR⁶R⁸, -C(O)R⁶, -OR⁶, -C(O)NR⁶R⁸, -OC(O)NR⁶R⁸, -NR⁸C(O)NR⁸R⁶, -NR⁸C(O)R⁶, -NR⁸C(O)O(C₁-C₄ alkyl), -C(NR⁸)NR⁸R⁶, -C(NCN)NR⁸R⁶, -C(NCN)S(C₁-C₄ alkyl), -NR⁸C(NCN)S(C₁-C₄ alkyl), -NR⁸C(NCN)NR⁸R⁶, -NR⁸SO₂(C₁-C₄ alkyl), -S(O)ₙ(C₁-C₄ alkyl) wherein n is 0 to 2, -NR⁸C(O)C(O)NR⁸R⁶, -NR⁸C(O)C(O)R⁸, thiazolyl, imidazolyl, oxazolyl, pyrazolyl, triazolyl, tetrazolyl, and C₁-C₄ alkyl optionally substituted by 1 to 3 fluoro substituents;
   each R⁶ and R⁷ is independently hydrogen or C₁-C₄ alkyl;
   each R⁸ is independently R⁶ or -OR⁶; and,
   each R⁹ is independently selected from cyano, R⁶, -OR⁶, -OC(O)R⁶, -C(O)OR⁶, -C(O)NR⁶R⁷, -NR⁶R⁷, -NR⁶R⁸, -SO₂NR⁶R⁷, and C₁-C₄ alkyl substituted by hydroxy; and
(b) compounds of the formula 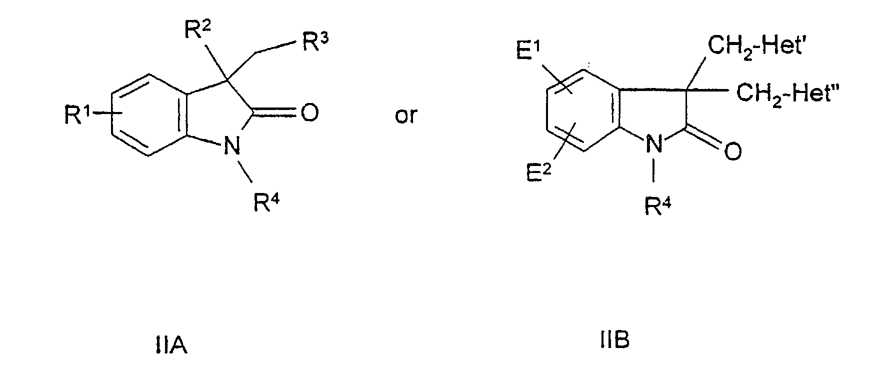 wherein R¹ is hydrogen, halo (e.g., chloro, fluoro, bromo or iodo), cyano, hydroxy, nitro, trifluoromethyl, -NHR⁵, -NR⁵R⁵, R⁵, -OR⁵ or -S(O)ₘ-R⁵;
   R² is -(CH₂)ₙ-Y or -OCOR⁵;
   R³ is 4-, 3-, or 2-pyridyl, pyrimidyl, pyrazinyl, 2-fluoro-4-pyridyl or 3-fluoro-4-pyridyl;
   R⁴ is 1-adamantyl or 2-adamantyl;
   Y is hydrogen, hydroxy, amino, cyano, -NHR⁵, -NR⁵R⁵, -NHCOR⁵, -NHCO₂R⁵, halo, OR⁵, -S(O)ₘR⁵, -CO₂H, -CO₂R⁵, -CONR⁵R⁵, -CONHR⁵, -CONH₂, -COR⁵, -CH=CHCO₂R⁵, -OCOR⁵, phenyl, phenyl substituted with W, -C≡CCO₂R⁵, -CH=CHR⁵ or -C≡CR⁵;
   each R⁵ is, independently, (C₁-C₄) straight or branched alkyl, phenyl or benzyl, wherein said phenyl and the phenyl moiety of said benzyl may optionally be substituted with halo, hydroxy, nitro, cyano, amino, (C₁-C₄) straight or branched alkyl, (C₁-C₄) straight or branched alkoxy, phenyl, benzyl, (C₁-C₄)alkylamino, di[(C₁-C₄)alkyl]amino, or -S(O)ₘ-(C₁-C₄) straight or branched alkyl;
   each W is, independently, halo, R⁵, hydroxy, -OR⁵, nitro, amino, -NHR⁵, -NR⁵R⁵, cyano, or -S(O)ₘ-R⁵;
   m is 0, or 2;
   n is 1 to 7;
   p is 0 or 1;
   E¹ and E² are selected, independently, from hydrogen, halo, (C₁-C₃)alkyl, hydroxy, (C₁-C₃)alkoxy, nitro, trifluoromethyl, cyano, amino, (C₁-C₃)alkylamino and di[(C₁-C₃)alkyl]amino;
   and their pharmaceutically acceptable salts.
   Het' and Het" are selected, independently, from 6 membered heterocyclic rings containing from one to four nitrogen atoms as part of the ring, optionally substituted with one substituent selected from (C₁-C₃)alkyl, halo, hydroxy, (C₁-C₃)alkoxy, amino, (C₁-C₃)alkylamino and di[(C₁-C₃)alkyl]amino; and
(c) compounds of the formula 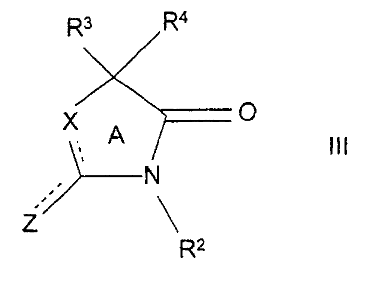 wherein both dotted lines represent optional double bonds;
   Z is oxygen or sulfur when it is double bonded to ring A and Z is hydroxy, (C₁-C₁₀)alkyl-S-, (C₁-C₁₀)alkyl-SO-, (C₁-C₁₀)alkyl-SO₂-, adamant-2-yl-S-, naphthyl-S-, benzyl-S-, phenyl-C(=O)CH₂-S-, (C₁-C₆)alkyl-O-C(=O)-CH₂-S- or (H,H) (i.e., Z represents two hydrogen atoms, each of which is single bonded to the same carbon of ring A) when Z is single bonded to ring A, and wherein said naphthyl and phenyl and the phenyl moiety of said benzyl may optionally be substituted with from one to three substituents independently selected from (C₁-C₆)alkyl optionally substituted with from one to three fluorine atoms, (C₁-C₆)alkoxy optionally substituted with from one to three fluorine atoms, halo (e.g., chloro, fluoro, bromo or iodo), amino, (C₁-C₆)alkylamino, [di-(C₁-C₆)alkyl]amino, cyano, nitro, (C₁-C₆)alkyl-SOₙ- wherein n is zero, one or two, -COOH, -COO(C₁-C₈)alkyl and -C(O)NH(C₁-C₆)alkyl;
   X is NR¹ or CHR¹;
   R¹ is hydrogen, (C₁-C₆)alkyl or (C₁-C₆)alkylphenyl when ring A is saturated (i.e., when ring A contains no double bonds) and R¹ is absent when ring A contains a double bond;
   R² is selected from naphthyl, phenyl, (C₁-C₆)alkylphenyl, 1-adamantyl, 2-adamantyl, (C₁-C₈) straight or branched alkyl, (C₃-C₁₀) cycloalkyl and (C₈-C₃₀)bicyclic or tricyclic alkyl; wherein said (C₃-C₁₀)cycloalkyl and said (C₈-C₃₀)bicyclic or tricyclic alkyl may optionally be substituted with a hydroxy group; and wherein said adamantyl groups may optionally be substituted with from one to three substituents independently selected from (C₁-C₆)alkyl, halo and hydroxy; and
   R³ and R⁴ are independently selected from benzyl, wherein the phenyl moiety of said benzyl may optionally be substituted with an amino or nitro group; hydrogen, phenyl, (N≡C)-(C₁-C₆)alkyl, (C₁-C₆)alkyl-O-C(=O)-(C₁-C₆)alkyl and Het-CH₂, wherein Het is selected from 2-, 3- or 4-pyridinyl, furyl, tetrahydrofuryl, pyrimidyl, pyrazinyl, pyrazolyl, isoxazolyl, thiophenyl and triazolyl;
   with the proviso that (a) no more than one of the two dotted lines can represent a double bond in any one compound, (b) when Z is (H, H), X is CH₂, (c) when Z is oxygen or (H, H) and X is CHR¹, R¹ must be hydrogen, (d) when Z is sulfur and X is NR¹, R¹ must be hydrogen, and (e) one of R³ and R⁴ must be Het-CH₂, and
(d) the compound 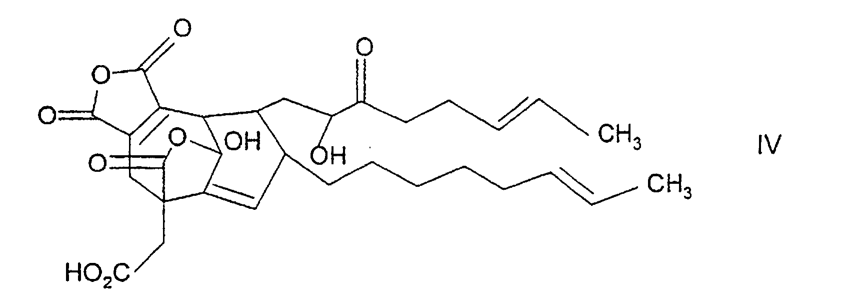 and the pharmaceutically acceptable salts of the foregoing compounds

Other more specific embodiments of this invention relate to any of the above pharmaceutical compositions and their use in the preparation of a medicament for methods of treatment wherein the FTase inhibitor is selected from compounds of the formula I as defined above, wherein R¹ and R² are both -(CH₂)ₚ(5-10 membered heterocycles) wherein p is 1 or 2.

Other more specific embodiments of this invention relate to any of the above pharmaceutical compositions and their use in the preparation of a medicament for methods of treatment wherein the FTase inhibitor is selected from compounds of the formula I as defined above, wherein R³ is a -(CH₂)ₘ(pinane) wherein m is 0, 1 or 2, and, more preferably, those wherein R³ is pinanemethyl.

Other more specific embodiments of this invention relate to any of the above pharmaceutical compositions and their use in the preparation of a medicament for methods of treatment wherein the FTase inhibitor is selected from compounds of the formula 1, as defined above, wherein R³ is 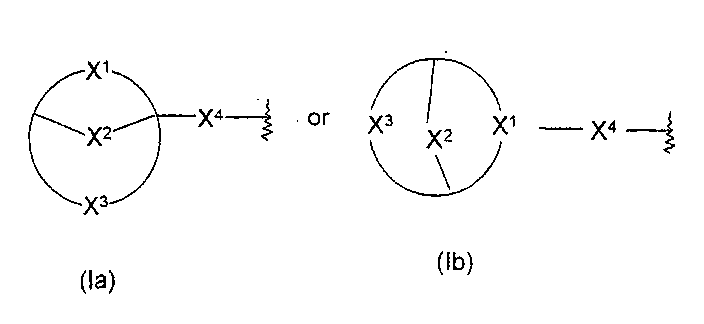 wherein X¹, X², X³ and X⁴ are as defined above.

Other more specific embodiments of this invention relate to any of the above pharmaceutical compositions and their use in the preparation of a medicament for methods of treatment wherein the FTase inhibitor is selected from compounds of the formula I, as described above, wherein R⁴ is phenyl optionally substituted by 1 to 3 R⁵ substituents.

Other more specific embodiments of this invention relate to any of the above pharmaceutical compositions and their use in the preparation of a medicament for methods of treatment, wherein the FTase inhibitor is selected from the compounds listed below:
2-[2-(4-Bromo-phenyl)-2-oxo-ethylidene]-5,5-bis-pyridin-4-ylmethyl-3-(2,66-trimethyl-bicyclo[3.1.1]hept-3-ylmethyl)-imidazolidin-4-one;
4-{[5-Oxo-4,4-bis-pyridin-4-ylmethyl-1-(2,6,6-trimethyl-bicyclo[3.1.1]hept-3-ylmethyl)-imidazolidin-2-ylidene]-acetyl}-benzonitrile;
2-[2-(4-Chloro-phenyl)-2-oxo-ethylidene]-5,5-bis-pyridin-4-ylmethyl-3-(2,6,6-trimethyl-bicyclo[3.1.1]hept-3-ylmethyl)-imidazolidin-4-one;
2-[2-(3,4-Dichloro-phenyl)-2-oxo-ethylidene]-55-bis-pyridin-4-ylmethyl-3-(2,66-trimethyl-bicyclo[3.1.1]hept-3-ylmethyl)-imidazolidin-4-one;
2-[2-(3-Nitro-phenyl)-2-oxo-ethylidene]-5,5-bis-pyridin-4-ylmethyl-3-(2,6,6-trimethyl-bicyclo[3.1.1]hept-3-ylmethyl)-imidazolidin-4-one;
2-[2-(4-Methoxy-phenyl)-2-oxo-ethylidene]-5,5-bis-pyridin-4-ylmethyl-3-(2,6,6-trimethyl-bicyclo[3.1.1]hept-3-ylmethyl)-imidazolidin-4-one;
2-[2-(3-Methoxy-phenyl)-2-oxo-ethylidene]-5,5-bis-pyridin-4-ylmethyl-3-(2,6,6-trimethyl-bicyclo[3.1.1]hept-3-ylmethyl)-imidazolidin-4-one;
2-[2-(2-Methoxy-phenyl)-2-oxo-ethylidene]-5,5-bis-pyridin-4-ylmethyl-3-(2,6,6-trimethyl-bicyclo[3.1.1]hept-3-ylmethyl)-imidazolidin-4-one;
2-(2-Biphenyl-4-yl-2-oxo-ethylidene)-5,5-bis-pyridin-4-ylmethyl-3-(2,6,6-trimethyl-bicyclo[3.1.1]hept-3-ylmethyl)-imidazolidin-4-one;
2-(2-Naphthalen-2-yl-2-oxo-ethylidene)-5,5-bis-pyridin-4-ylmethyl-3-(2,6,6-trimethyl-bicyclo[3.1.1]hept-3-ylmethyl)-imidazolidin-4-one;
2-[2-(4-Fluoro-phenyl)-2-oxo-ethylidene]-5,5-bis-pyridin-4-ylmethyl-3-(2,6,6-trimethyl-bicyclo[3.1.1]hept-3-ylmethyl)-imidazolidin-4-one;
2-[2-(2,4-Difluoro-phenyl)-2-oxo-ethylidene]-5,5-bis-pyridin-4-ylmethyl-3-(2,6,6-trimethyl-bicyclo[3.1.1]hept-3-ylmethyl)-imidazolidin-4-one;
4-{[5-Oxo-4,4-bis-pyridin-4-ylmethyl-1-(2,6,6-trimethyl-bicyclo[3.1.1]hept-3-yl)-imidazolidin-2-ylidene]-acetyl}-benzonitrile;
2-[2-(4-Nitro-phenyl)-2-oxo-ethylidene]-5,5-bis-pyridin-4-ylmethyl-3-(2,6,6-trimethyl-bicyclo[3.1.1]hept-3-ylmethyl)-imidazolidin-4-one;
2-[2-Oxo-2-phenyl-ethylidene]-5,5-bis-pyridin-4-ylmethyl-3-(2,6,6-trimethyl-bicyclo[3.1.1]hept-3-ylmethyl)-imidazolidin-4-one;
2-{2-Oxo-2-[4-(2H-tetrazol-5-yl)-phenyl]-ethylidene}-5,5-bis-pyridin-4-ylmethyl-3-(2,66-trimethyl-bicyclo[3.1.1]hept-3-ylmethyl)-imidazolidin-4-one;
3-{[5-Oxo-4,4-bis-pyridin-4-ylmethyl-1-(2,6,6-trimethyl-bicyclo[3.1.1]hept-3-ylmethyl)-imidazolidin-2-ylidene]-acetyl}-benzonitrile;
4-{[5-Oxo-4,4-bis-pyridin-4-ylmethyl-1-(2,6,6-trimethyl-bicyclo[3.1.1]hept-3-ylmethyl)-imidazolidin-2-ylidene]-acetyl}-benzoic acid ethyl ester;
2-[2-Oxo-2-(4-trifluoromethyl-phenyl)-ethylidene]-5,5-bis-pyridin-4-ylmethyl-3-(2,6,6-trimethyl-bicyclo[3.1.1]hept-3-ylmethyl)-imidazolidin-4-one;
2-[2-(4-Methanesulphonyl-phenyl)-2-oxo-ethylidene]-5,5-bis-pyridin-4-ylmethyl-3-(2,6,6-trimethyl-bicyclo[3.1.1]hept-3-ylmethyl)-imidazolidin-4-one;
4-{[1-(6,6-Dimethyl-bicyclo[3.1.1]hept-2-ylmethyl)-5-oxo-4,4-bis-pyridin-4-ylmethyl-imidazolidin-2-ylidene]-acetyl}-benzonitrile;
4-[(1-Bicyclo[22.2]oct-1-ylmethyl-5-oxo-4,4-bis-pyridin-4-ylmethyl-imidazolidin-2-ylidene)-acetyl]-benzonitrile;
4-{[1-(2-Ethyl-6,6-dimethyl-bicyclo[3.1.1]hept-3-ylmethyl)-5-oxo-4,4-bis-pyridine-ylmethyl-imidazolidin-2-ylidene]-acetyl}-benzonitrile;
4-{[1-(2-Benzyl-6,6-dimethyl-bicyclo[3.1.1]hept-3-ylmethyl)-5-oxo-4,4-bis-pyridin-4-ylmethyl-imidazolidin-2-ylidene]-acetyl}-benzonitrile;
4-{[1-(2-lsopropenyl-6,6-dimethyl-bicyclo[3.1.1]hept-3-ylmethyl)-5-oxo-4,4-bis-pyridin-4-ylmethyl-imidazolidin-2-ylidene]-acetyl}-benzonitrile;
4-{[1-(2-Isopropyl-6,6-dimethyl-bicyclo[3.1.1]hept-3-ylmethyl)-5-oxo-4,4-bis-pyridin-4-ylmethyl-imidazolidin-2-ylidene]-acetyl}-benzonitrile;
4-({1-[2-(1-Methoxyimino-ethyl)-6,6-dimethyl-bicyclo[3.1.1]hept-3-ylmethyl]-5-oxo-4,4-bis-pyridin-4-ylmethyl-imidazolidin-2-ylidene}-acetyl)-benzonitrile;
4-{[1-(6,6-Dimethyl-2-methylene-bicyclo[3.1.1]hept-3-ylmethyl)-5-oxo-4,4-bis-pyridin-4-ylmethyl-imidazolidin-2-ylidene]-acetyl}-benzonitrile;
4-{[1-(2-Hydroxy-2-hydroxymethyl-6,6-dimethyl-bicyclo[3.1.1]hept-3-ylmethyl)-5-oxo-4,4-bis-pyridin-4-ylmethyl-imidazolidin-2-ylidene]-acetyl}-benzonitrile;
4-{[1-(6,6-Dimethyl-2-oxo-bicyclo[3.1.1]hept-3-ylmethyl)-5-oxo-4,4-bis-pyridin-4-ylmethyl-imidazolidin-2-ylidene]-acetyl}-benzonitrile;
3-tert-Butyl-2-(2-oxo-2-phenyl-ethylidene)-5,5-bis-pyridin-4-ylmethyl-imidazolidin-4-one;
4-{[1-(2,2-Dimethyl-propyl)-5-oxo-4,4-bis-pyridin-4-ylmethyl-imidazolidin-2-ylidene]-acetyl}-benzonitrile;
4-{[1-(2-Adamantan-1-yl-ethyl)-5-oxo-4,4-bis-pyridin-4-ylmethyl-imidazolidin-2-ylidene]-acetyl}-benzonitrile;
3-Cyclohexyl-2-(2-oxo-2-phenyl-ethylidene)-5,5-bis-pyridin-4-ylmethyl-imidazolidin-4-one;
4-{(1-Adamant-1-ylmethyl-5-oxo-4,4-bis-pyridin-4-ylmethyl-imidazolidin-2-ylidene)-acetyl]-benzonitrile;
4-[(1-Cyclohexylmethyl-5-oxo-4,4-bis-pyridin-4-ylmethyl-imidazolidin-2-ylidene)-acetyl]-benzonitrile;
3-Hexyl-2-(2-oxo-2-phenyl-ethylidene)-5,5-bis-pyridin-4-ylmethyl-imidazolidin-4-one;
3-Napthalen-1-yl-2-(2-oxo-2-phenyl-ethylidene)-5,5-bis-pyridin-4-ylmethyl-imidazolidin-4-one;
3-Adamantan-1-yl-2-(2-oxo-2-phenyl-ethylidene)-5,5-bis-pyridin-4-ylmethyl-imidazolidin-4-one;
3-Adamantan-1-yl-2-[2-(4-nitro-phenyl)-2-oxo-ethylidene]-5,5-bis-pyridin-4-ylmethyl-imidazolidin-4-one;
4-[(1-Benzyl-5-oxo-4,4-bis-pyridin-4-ylmethyl-imidazolidin-2-ylidene)-acetyl]-benzonitrile;
4-[(1-Allyl-5-oxo-4,4-bis-pyridin-4-ylmethyl-imidazolidin-2-ylidene)-acetyl]-benzonitrile;
4-[(1-Methyl-5-oxo-4,4-bis-pyridin-4-ylmethyl-imidazolidin-2-ylidene)-acetyl]-benzonitrile;
4-{[1-(2,2-Diethoxy-ethyl)-5-oxo-4,4-bis-pyridin-4-ylmethyl-imidazolidin-2-ylidene]-acetyl}-benzonitrile;
4-[(1-Adamantan-2-ylmethyl-5-oxo-4,4-bis-pyridin-4-ylmethyl-imidazolidin-2-ylidene)-acetyl]-benzonitrile;
4-[(1-Adamantan-2-yl-5-oxo-4,4-bis-pyridin-4-ylmethyl-imidazolidin-2-ylidene)-acetyl]-benzonitrile;
4-[(5-Oxo-1-phenyl-4,4-bis-pyridin-4-ylmethyl-imidazolidin-2-ylidene)-acetyl]-benzonitrile; and,
4-{[4-tert-Butyl-phenyl-5-oxo-4,4-bis-pyridin-4-ylmethyl-imidazolidin-2-ylidene)-acetyl]-benzonitrile.
and the pharmaceutically acceptable salts of such compounds.

Other more specific embodiments of this invention relate to any of the above pharmaceutical compositions and their use in the preparation of a medicament for methods of treatment wherein the HMG CoA reductase inhibitor contained in such composition. or used in such method is selected from the group consisting of atorvastatin, pravastatin, niacin, gemfibrozil, clofibrate, lovastatin, fluvastatin, simvastatin and compactin, and the pharmaceutically acceptable salts of the foregoing compounds.

Other more specific embodiments of this invention relate to any of the above pharmaceutical compositions and their use in the preparation of a medicament for methods of treatment wherein the HMG CoA reductase inhibitor contained in such composition or used in such method is atorvastatin.

Other more specific embodiments of this invention relate to any of the above pharmaceutical compositions and their use in the preparation of a medicament for methods of treatment wherein the HMG CoA reductase inhibitor contained in such composition or used in such method is lovastatin.

Other more specific embodiments of this invention relate to any of the above the pharmaceutical compositions and their use in the preparation of a medicament for methods of treatment wherein the FTase inhibitor contained in such composition or used in such method is selected from:
(a) compounds of the formula IIA, as defined above, wherein R³ is 4-pyridyl, 4-pyrimidyl or 2-fluoro-4-pyridyl;
(b) compounds of the formula IIA, as defined above, wherein R² is -(CH₂)ₙY;
(c) compounds of the formula IIA, as defined above, wherein R² is -(CH₂)ₙY and n is an integer from 1 to 5;
(d) compounds of the formula IIA, or IIB as defined above, wherein each of R¹, E¹, E² and R⁴, if present, is hydrogen; and
(e) compounds of the formula IIA, as defined above, wherein R² is -(CH₂)ₙ-Y, R¹ is 4-pyridyl, 4-pyrimidyl or 2-fluoro-4-pyridyl, R⁵ is (C₁-C₂) alkyl and Y is -CO₂R⁵, cyano, -CONHR⁴, CH=CHCO₂R⁵ or -OCOR⁵;

Other more specific embodiments embodiments of this invention relate to any of the above pharmaceutical compositions and their use in the preparation of a medicament for methods of treatment wherein the FTase inhibitor contained in such composition or used in such method is not limonene or d-limonene.

The term "alkyl", as used herein, unless otherwise indicated, includes saturated monovalent hydrocarbon radicals having straight, branched or cyclic moieties or combinations thereof.

The term "halo", as used herein, refers to chloro, fluoro, bromo or iodo.

The above compounds of the formulas I, IIA, IIB, III and IV may contain one or more chiral centers and therefore may exist in 2 or more enantiomeric and diastereomeric forms. The above definitions of the compounds having formulas I, IA, IIB, III and IV include all enantiomers, diasteriomers and other stereoisomers of these compounds, as well as mixtures thereof.

The following references refer to compounds that exhibit activity as FTase inhibitors and which can be used, in combination with an HMG CoA reductase inhibitor, in the pharmaceutical compositions and methods of this invention, and to methods of preparing the same: International Patent Application PCT/US92/11292, which designates the United States and was published on July 22, 1993 as WO 93/14085; United States Patent 4,876,259, which issued on October 24, 1989; United States Patent H1345 which issued on August 2, 1994; United States Patent 5,260,332, which issued on November 9, 1993; United States Patent 5,262,435, which issued on November 16, 1993; United States Patent 5,369,125, issued on November 29, 1994; World Patent Application WO 93/24633, which was published on December 9, 1993: World Patent Application WO 94/03597, which was published on February 17, 1994; World Patent Application WO 94/16069, which was published on June 21,1994; G.L. Bulton, et al., 209th American Chem. Soc. Nat'l Meeting, Anaheim, Ca, April 2-6, 1995, Division of Med. Chem., Abs. No. 032, World Patent Application WO 95/00497, which was published on January 5, 1995; United States Patent 5,260,479, which was published on November 9, 1993; World Patent Application WO 95/10514; World Patent Application WO 95/10515; World Patent Application WO 95/10516; World Patent Application WO 95/12572, which was published on May 11, 1995; World Patent Application WO 95/11917, which was published on May 4, 1995; World Patent Application WO 94/26723, which was published on November 24, 1994; World Patent Application WO 95/25086, which was published on September 21, 1995; Kanda et al, AFMC. International Medicinal Chemistry Symposium AIMECS 95, Tokyo, Japan, Poster, P7M153, Sept. 4, 1995; World Patent Application WO 96/10037 which was published on April 4, 1996; World patent Application 96/10035, which was published on April 4, 1996; World Patent Application WO 96/10034, which was published on April 4, 1996; World Patent Application WO 96/10011, which was published on Apri 6, 2996; World Patent Application WO 96/10011, which was published on April 6, 1996; World Patent Application WO 96/09821, which was published on April 4, 1996; World Patent Application WO 96/09820, which was published on April 4, 1996; Quin et al, 211th American Chemical Society National Meeting, New Orleans, La., March 24-28, 1996, Lecture, COMP 012, March 24, 1996; World Patent Applications WO 96/06609 and WO 96/06604, both of which were published on March 7, 1996; European Patent Application EP 696,593, which was published on February 14, 1996; Hartman, G. D., 14th International Symposium on Medicinal Chemistry, Maastricht, Netherlands, September 8-12, 1996, Lectura, SL-08.3, Sept. 10, 1996; World Patent Application WO 96/30363, which was published on October 3, 1996; World Patent Application WO 96/30343, which was published in October 3, 1996, World Patent Application WO 97/03050; World Patent Application WO 94/26723, which was published on November 24, 1994; International Patent Application PCT/IB95/00189, which designates the United States and was filed on March 20 1995; United States Patent Application 08/236,743, which was filed on April 29, 1994; United States Provisional Application entitled "Adamantyl Substituted Oxindoles As Pharmaceutical Agents," which was filed on May 28, 1996, in the name of R.A. Volkmann and J.P. Lyssikatos; United States Patent 5,350,867, which issued on September 27, 1994; United States Patent 5,352,705, which issued on October 4, 1994; United States Patent 5,565,489, which issued on October 15, 1996; European Patent Application EP 750,609, which was published on January 2, 1997; European Patent Application 461,869, which was published on December 18, 1991; and World Patent Application 96/21456, which was published on July 18, 1996.

The following references refer to compounds that exhibit activity as HMG CoA reductase inhibitors and which can be used, in combination with a FTase inhibitor, in the pharmaceutical compositions and methods of this invention, and to methods of preparing the same: United States Patent 4,681,893, issued July 21, 1987; United States Patent 5,273,995, issued December 28, 1993; United States Patent 5,385,929, issued January 31, 1995; United States Patent 4,957,971, issued September 18, 1990; United States Patent 5,102,893, issued April 7, 1992; United States i Patent 4,957,940, issued September 18, 1990; United States Patent 4,950,675, issued August 21, 1990; United States Patent 4,929,620, issued May 29, 1990; United States Patent 4,923,861, issued May 8, 1990; United States Patent 4,906,657, issued March 6, 1990; United States Patent 4,868,185, issued September 19, 1989; United States Patent 5,124,482 issued June 23, 1992; United States Patent 5,003,080, issued March 26, 1991; United States Patent 5,097,045, issued March 17, 1992; United States Patent 5,149,837, issued September 22, 1992; United States Patent 4,906,624, issued March 6, 1990; United States Patent 4,761,419, issued August 2, 1988; United States Patent 4,735,950, issued April 5, 1988; United States Patent 4,808,621, issued February 28, 1989; United States Patent 4,647,576, issued March 3, 1987; United States Patent 5,118,882, issued June 2, 1992; United States Patent 5,214,197, issued May 25, 1993; United States Patent 5,321,046, issued June 14, 1994; United States Patent 5,260,440, issued . November 9, 1993; and United States Patent 5,208,258 issued May 4, 1993; United States Patent 5,369,125, issued November 29, 1994; United States Patent H1345 issued August 2, 1994; United States Patent 5,262,435, issued November 16, 1993; and United States Patent 5,260,332, issued November 9, 1993. Great Britian Patent Application GB 2,055,100, published February 25, 1981; United States Patent 4,499,289, issued February 12, 1983; United States Patent 4,645,854, issued February 24, 1987; United States Patent 4,613,610 issued September 23, 1986; United States Patent 4,668,699, issued May 26, 1987; United States Patent 4,851,436, issued July 25, 1989; United States Patent 4,678,806, issued July 7, 1987; United States Patent 4,772,626, issued September 20, 1988; United States Patent 4,855,321, issued August 8, 1989; European Patent Application EP 244364, published November 4, 1987; United States Patent 4,766,145, issued August 23, 1988; United States Patent 4,876,279, issued October 24, 1989; United States Patent 4,847,306, issued July 11, 1989; United States Patent 5,049,696, issued September 17, 1991; European Patent Application EP 245,990, published November 19, 1987; European Patent Application EP 251,625, published January 7, 1988; United States Patent 4,719,229, published January 12, 1988; Japanese Patent Application 63014722, published January 21, 1988; United States Patent 4,736,064, issued April 5, 1988; United States Patent, 4,738,982 issued April 19, 1988; United States Patent 4,845,237, issued July 4, 1989; European Patent EP 306,263, granted March 18, 1992; United States Patent 5,026,708, issued June 25, 1991; United States Patent 4,863, 957, issued September 5, 1989; United States Patent 4,946,841, issued August 7, 1990; European Patent 339358, granted July 13, 1994; United States Patent 4,937,264 issued June 26, 1998; United States Patent 4,876,366, issued October 24, 1989; United States Patent 4,921,974, issued May 1, 1990; United States Patent 4,963,538 issued October 16, 1990; United States Patent 5,130,306, issued July 14, 1992; United States Patent 4,900,754 issued February 13, 1990; United States Patent 5,026,698, issued June 25, 1991; United States Patent 4,977,161, issued December 11, 1990; United States Patent 4,927,851, issued May 22, 1990; European Patent Application EP 373,507, published June 20, 1990; United States Patent 4,939,143, issued July 3, 1990; United States Patent 4,939,159, issued July 3, 1990; United States Patent 4,940,727, issued July 10, 1990; United States Patent 5,116,870, issued May 26, 1992; Australian Patent AU 635,545, granted March 25, 1993; United States Patent 5,098,391, issued March 24, 1992; United States Patent 5,294,724, issued March 15, 1994; United States Patent 5,001,255, issued March 19, 1991; United States Patent 5,149,834, issued September 22, 1992; United States Patent 5,089,523, issued February 18, 1992; European Patent Application EP 465,265 published January 8, 1992; United States Patent 5,476,846, issued December 19, 1995; United States Patent 5,321,046, issued June 14, 1994; United States Patent 5,106,992, issued April 21, 1992; United States Patent 5,347,039, issued September 13, 1994; Japanese Patent Application 4193836, published July 13, 1992; Great Britian patent Application 2253787, published September 23, 1992; United States Patent 5,411,969, issued May 2, 1995; Japanese Patent Application 4,356,435, published December 10, 1992; United States Patent 5,266,707 issued November 30, 1993; United States Patent 5,455,247 issued October 3, 1995; United States Patent 5,475,029, issued December 12, 1995; United States Patent 5,591,772, issued January 7, 1997; United States Patent 5,286,746 issued February 15, 1994; Japanese Patent Application JP 7089898, published April 4, 1995; European Patent Application EP 677,039, published October 18, 1995 and World Patent Application 96108248, published March 21, 1996.

This invention relates both to the preparation of a medicament for treating cancer in which the FTase inhibitor and the HMG CoA reductase inhibitor are to be administered together, as part of the same pharmaceutical composition, as well as the case in which these two active agents are to be administered separately as part of an appropriate dose regimen designed to obtain the benefits of the combination therapy. The appropriate dose regimen, the amount of each dose administered, and specific intervals between doses of each active agent will depend upon the subject being treated, the type of cancer or abnormal cell growth and the severity of the condition. In carrying out the invention, the FTase inhibitor will be administered in the amounts disclosed in the literature, or otherwise believed to be effective, for the administration of such compound as a single active agent for the treatment of cancer or the inhibition of abnormal cell growth, and the HMG CoA reductase inhibitor will be administered in an amount that is about one quarter to one half of the amount disclosed in the literature, or otherwise believed to be effecive, for administration of such compound as a single agent for the treatment of hypercholesterolemia. For example, in carrying out the present inventions, the FTase inhibitors of formulas I, IIA, IIB and III will typically be admisterered to an average 70 kg adult human in an amount ranging from about 0.005 to about 0.6 mg per kg body weight of the subject being treated per day, in single or divided doses, and the HMG CoA reductase inhibitor atorvastatin will typically be administered in an amount ranging from about 0.07 to about 3.6 mg per kg body weight per day, in single or divided doses. Variations may nevertheless occur depending upon the species of animal being treated and its individual response to said medicament, as well as on the type of pharmaceutical formulation chosen and the time period and interval at which such administration is carried out. In some instances, dosage levels below the lower limit of the above range may be more than adequate, while in other cases dosage levels higher than the above upper daily limit may be employed without causing any harmful side effect, provided that such larger dosages are administered as several small doses for administration throughout the day.

The FTase inhibitors and the HMG CoA reductase inhibitors that are employed in the pharmaceutical compositions of this invention are hereinafter also referred to as "therapeutic agents". The therapeutic agents can be administered via either the oral or parenteral route. Compositions containing both a FTase inhibitor and an HMG CoA reductase inhibitor will generally be administered orally or parenterally daily, in single or divided doses, so that the total amount of each active agent to be administered falls within the above guidelines.

The therapeutic agents may be administered alone or in combination with pharmaceutically acceptable carriers or diluents by either of the routes previously indicated, and such administration may be carried out in single or multiple doses. More particularly, the novel therapeutic agents of this invention can be administered in a wide variety of different dosage forms, i.e., they may be combined with various pharmaceutically acceptable inert carriers in the form of tablets, capsules, lozenges, troches, hard candies, suppositories, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the therapeutic compounds of this invention, when administered separately (i.e., not in the same pharmaceutical composition) are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also-be employed as fillers in gelatin capsules; preferred materials in this connection also include lactose or milk sugar as well as high molecular weight polyethylene glycols. When aqueous suspensions and/or elixirs are desired for oral administration. the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a therapeutic agent in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

The activity of the therapeutic compounds as FTase inhibitors may be determined by their ability, relative to a control, to inhibit Ftase in vitro. This procedure is described below.

A crude preparation of FTase comprising the cytosolic fraction of homogenized brain tissue is used for screening compounds in a 96-well assay format. The cytosolic fraction is prepared by homogenizing approx. 40 grams fresh tissue in 100 ml of sucrose/MgCl₂/EDTA buffer (using a Dounce homogenizer; 10-15 strokes), centrifuging the homogenates at 1000 grams for 10 minutes at 4G, re-centrifuging the supernatant at 17,000 grams for 15 minutes at 4G, and then collecting the resulting supernatant. This supernatant is diluted to contain a final concentration of 50 mM Tris HCI (pH 7.5), 5 mN DTT, 0.2 M KCI, 20 mM ZnCl₂, 1 mM PMSF and re-centrifuged at 178,000 grams for 90 minutes at 4G. The supernatant, termed "crude FTase" was assayed for protein concentration, aliquoted, and stored at -70°C.

The assay used to measure in vitro inhibition of human FTase is a modification of the method described by Amersham LifeScience for using their Farnesyl transferase (3H) Scintilation Proximity Assay (SPA) kit (TRKQ 7010). FTase enzyme activity is determined in a volume of 100 ml containing 50 mM N-(2-hydroxy ethyl) piperazine-N¢-(2-ethane sulfonic acid) (HEPES), pH 7.5, 30 mM MgCl₂, 20 uM KCI, 5 mM Na₂HPO₄, 5 mM dithiothreitol (DTT), 0.01% Triton X-100, 5% dimethyl sulfoxide (DMSO), 20 mg of crude FTase, 0.12 mM [3H]-farnesyl pyrophosphate ([3H]-FPP; 36000 dpm/pmole, Amersham LifeScience), and 0.2 mM of biotinylated Ras peptide KTKCVIS (Bt-KTKCVIS) that is N-terminally biotinylated at its alpha amino group and was synthesized and purified by HPLC in house. The reaction is initiated by addition of the enzyme and terminated by addition of EDTA (supplied as the STOP reagent in kit TRKQ 7010) following a 45 minute incubation at 37°C. Prenylated and unprenylated Bt-KTKCVIS is captured by adding 10 ml of steptavidin-coated SPA beads (TRKQ 7010) per well and incubating the reaction mixture for 30 minutes at room temperature. The amount of radioactivity bound to the SPA beads is determined using a MicroBeta 1450 plate counter. Under these assay conditions, the enzyme activity is linear with respect to the concentrations of the prenyl group acceptor, Bt-KTKCVIS, and crude FTase, but saturating with respect to the prenyl donor, FPP. The assay reaction time is also in the linear range.

The test compounds are routinely dissolved in 100% DMSO. Inhibition of farnesyl transferase activity is determined by calculating percent incorporation of tritiated-farnesyl in the presence of the test compound vs. its incorporation in control wells (absence of inhibitor). IC₅₀ values, that is, the concentration required to produce half maximal farnesylation of Bt-KTKCVIS, is determined from the dose-responses obtained.

A fluorsecence assay for FTase activity that can be used to screen for FTase inhibitors is described in UK Patent Application GB 2,267,966, which was published on December 22, 1993.

The activity of certain therapeutic agents as HMG CoA reductase inhibitors may be determined by the procedure described by Dugan et al, Achiv. Biochem. Biophys., (1972), 152, 21-27. In this method, the level of HMG-CoA enzyme activity in standard laboratory rats is increased by feeding the rats a chow diet contining 5% cholestyramine for four days, after which the rats are sacrificed. The rat livers are homogenized, and the incorporation of cholesterol-¹⁴C-acetate into nonsaponifiable lipid by the rat liver homogenate is measured. The micromolar concentration of compound required for 50% inhibition of sterol synthesis over a one-hour period is measured, and expressed as an IC₅₀ value.

A second method (designated COR screen) is that described by T. Kita, et al, J. Clin. Invest., (1980), 66: 1094-1100. In this method, the amount of ¹⁴C-HMG-CoA converted to ¹⁴C-mevalonate in the presence of a purified enzyme preparation of HMG-CoA reductase is measured. The micromolar concentration of compound required for 50% inhibition of cholesterol synthesis is measured and recorded as an IC₅₀ value.

The invention may be useful for a therapy that includes the administration of one or more other anti-tumor substances, for example, those selected from mitotic inhibitors, for example, vinblastine; alkylating agents, for example, cisplatin, carboplatin and cyclophosphamide; antimetabolites, for example, 5-fluorouracil, cystosine arabinoside and hydroxyurea, or, for example, one of the preferred antimetabolites disclosed in European Patent Application No. 239362 such as N-{5-[N-(3,4-dihydro-2-methyl-4-oxoquinazolin-6-ylmethyl)-N-methylamino]-2-thenoyl}-L-glutamic acid; intercalating antibiotics, for example, adriamycin and bleomycin; enzymes, for example, asparaginase; topoisomerase inhibitors, for example, etoposide; biological response modifiers, for example, interferon; and anti-hormones, for example, antioestrogens such as 'NOLVADEX' (tamoxifen) or antiandrogens such as 'CASODEX' (4'-cyano-3-(4-fluorophenylsulphonyl)-2-hydroxy-2-methyl-3'-(trifluoromethyl)propionanilide. Such therapies may be achieved by way of the simultaneous, sequential or separate dosing of the individual components of the therapy. According to this aspect of the invention, there is provided a pharmaceutical product comprising a pharmaceutically acceptable carrier, as described above, one or both of an HMG CoA reductase inhibitor and a FTase inhibitor, and an additional anti-tumor agent, as described above.

As indicated in Table 1 below, the present inventor has shown that the effectiveness of Compound 1, which has the structure 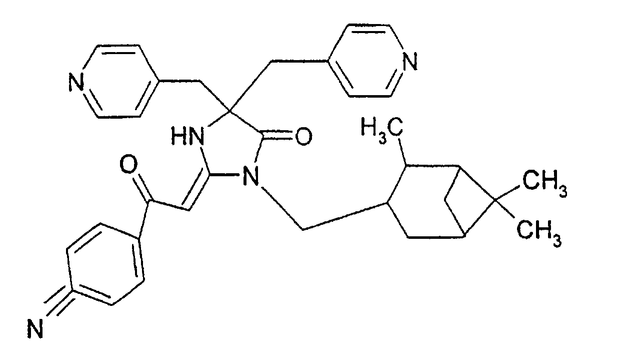 can be enhanced by a minimally effective dose of lovastatin.

**TABLE 1**

| Synergistic Effects of Lovastatin and Compound 1 Treatment on Prenylation of K-*ras* 4B in Intact Cells | | |
|---|---|---|
| | % Inhibition OF K-Ras 4B Prenylation* | |
| Compound 1[µm] | CONTROL | + 5µM Lovastatin |
| 0 | 0 | 23 |
| 0.1 | 0 | 56 |
| 1.0 | 0 | 83 |
| 10 | 0 | 96 |

| | | |
|---|---|---|
| * Semi-confluent monolayers of the NIH-3T3 tranfectant overexpressing mutant K-Ras 4B were treated for 18 hours at 37C with increasing concentrations of CP-390,392 in the presence and absence of 5 µM of hydrolysed lovastatin. Cells were lysed in a RIPA lysis buffer (50 mM tris[hydroxymethyl] amino-methane, 0.15M sodium chloride, 1% sodium deoxycholate, 1% Triton X- 100, 0.1% SDS, 0.25 sodium azide; ph 8.5) containing 1 mM of DTT (dithiothreitol; Boehringer Mannheim, Indianapolis, IN) and protease inhibitors (Aprotinin, Leupeptin, Anitpain, Pefabloc at final concentrations of 10 µg/ml, 2 µg/ml, 2 µg/ml and 50 µM, respectively; Boehringer Mannheim, Indianapolis, IN) and boiled for 3 minutes. Equal amounts of protein (100 µg/lane) were resolved by SDS-PAGE on 12.5% gels and transferred to Immobilon-P membranes (Intergrated Separation Systems, Natick, MA.). The membranes were immunoblotted with 5 µg/ml of anti-Pan-*ras* (Ab-3) monoclonal antibody (Calbiochem, La Jolla, CA). The blots were incubated with peroxidase-conjugated secondary antibody, and the immunoblotted *Ras* protein were detected by enhanced chemiluminescence (Amersham Life Products, Arlington Heights, IL). Percent of prenylated *Ras* was determined by densitometric scanning using MasterScan 3.0 (Scanalytics, Billerica, Massachusettes). | | |

## Claims

1. A pharmaceutical composition for the treatment of cancer or a benign proliferative disorder in a mammal, comprising an FTase inhibitor, an HMG CoA reductase inhibitor and a pharmaceutically acceptable carrier, wherein the FTase inhibitor and the HMG CoA reductase inhibitor are present in amounts that render the composition effective in the treatment of cancer or a benign proliferative disorder and wherein the FTase inhibitor is selected from:
(a) compounds of the formula I 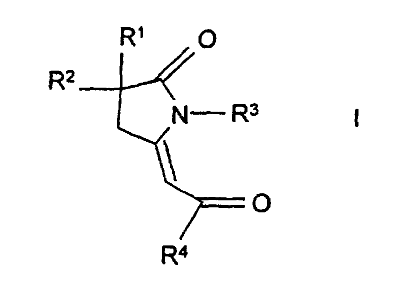 wherein:
R¹ and R² are independently selected from the group consisting of -(CH₂)ₚ(5-10 membered heterocycles), -(CH₂)ₚ(C₆-C₁₀ aryl), allyl, propargyl and C₁-C₆ alkyl wherein p is 0 to 3, said alkyl and the alkyl moieties of said R¹ and R² groups are optionally substituted by 1 to 3 R⁹ substituents, and the aryl and heterocyclic moieties of said R¹ and R² groups are optionally substituted by 1 to 3 substituents independently selected from halo and R⁹;
R³ is -(CH₂)ₘ(1- or 2-adamantyl), -(CH₂)ₘ(C₃-C₁₀ cycloalkyl), -(CH₂)ₘ(C₆-C₁₀ aryl), C₁-C₁₀ alkyl, 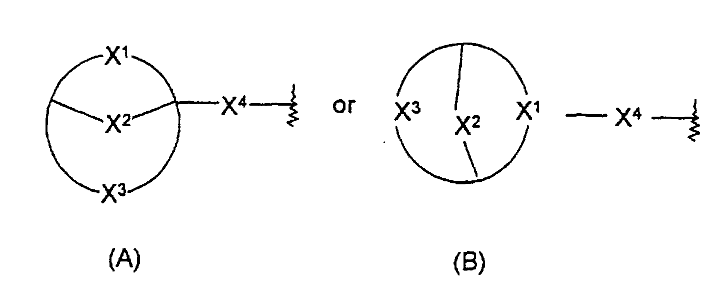
wherein m is 0 to 6, and said cycloalkyl and alkyl optionally contain 1 or 2 double or triple bonds;
X¹, X², and X³ are each independently C₁-C₇ alkylene optionally containing 1 or 2 double or triple bonds, X⁴ is a bond or C₁-C₇ alkylene optionally containing 1 or 2 double or triple bonds, and, in formula (B), the X⁴ moiety is attached to the X¹ moiety at any available carbon in the X¹ moiety;
R⁴ is C₆-C₁₀ aryl, 5-10 membered heterocyclyl or C₁-C₆ alkyl wherein each of said R⁴ groups is optionally substituted by 1 to 3 R⁵ substituents;
each R⁵ is independently selected from the group consisting of halo, nitro, cyano, phenyl, -C(O)OR⁶, -SO₂NR⁶R⁷, -NR⁶R⁸, -C(O)R⁶, -OR⁶, -C(O)NR⁶R⁸, -OC(O)NR⁶R⁸, -NR⁸C(O)NR⁸R⁶, -NR⁸C(O)R⁶, -NR⁸C(O)O(C₁-C₄ alkyl), -C(NR⁸)NR⁸R⁶, -C(NCN)NR⁸R⁶, -C(NCN)S(C₁-C₄ alkyl), -NR⁸C(NCN)S(C₁-C₄ alkyl), -NR⁸C(NCN)NR⁸R⁶, -NR⁸SO₂(C₁-C₄ alkyl), -S(O)ₙ(C₁-C₄ alkyl) wherein n is 0 to 2, -NR⁸C(O)C(O)NR⁸R⁶, -NR⁸C(O)C(O)R⁸, thiazolyl, imidazolyl, oxazolyl, pyrazolyl, triazolyl, tetrazolyl, and C₁-C₄ alkyl optionally substituted by 1 to 3 fluoro substituents;
each R⁶ and R⁷ is independently hydrogen or C₁-C₄ alkyl;
each R⁸ is independently R⁶ or -OR⁶; and,
each R⁹ is independently selected from cyano, R⁶, -OR⁶, -OC(O)R⁶, -C(O)OR⁶, -C(O)NR⁶R⁷, -NR⁶R⁷, -NR⁶R⁸, -SO₂NR⁶R⁷, and C₁-C₄ alkyl substituted by hydroxy;
(b) compounds of the formula IIA or IIB 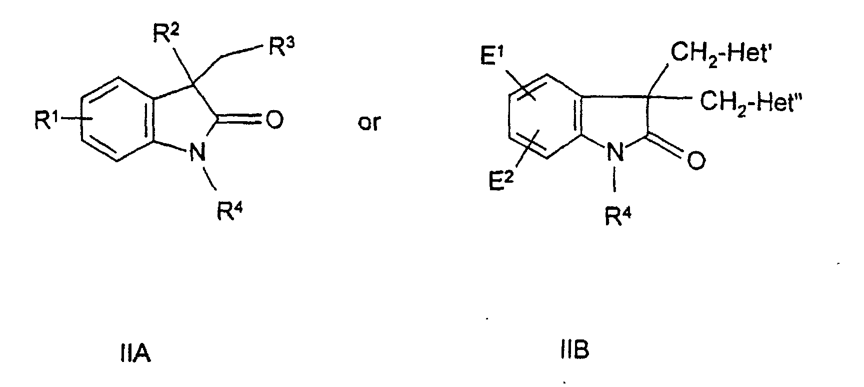 wherein:
R¹ is hydrogen, halo (e.g., chloro, fluoro, bromo or iodo), cyano, hydroxy, nitro, trifluoromethyl, -NHR⁵, -NR⁵R⁵, R⁵, -OR⁵ or -S(O)ₘ-R⁵;
R² is -(CH₂)ₙ-Y or -OCOR⁵;
R³ is 4-, 3-, or 2-pyridyl, pyrimidyl, pyrazinyl, 2-fluoro-4-pyridyl or 3-fluoro-4-pyridyl;
R⁴ is 1-adamantyl or 2-adamantyl;
Y is hydrogen, hydroxy, amino, cyano, -NHR⁵, -NR⁵R⁵, -NHCOR⁵, -NHCO₂R⁵, halo, OR⁵, -S(O)ₘR⁵, -CO₂H, -CO₂R⁵, -CONR⁵R⁵, -CONHR⁵, -CONH₂, -COR⁵, -CH=CHCO₂R⁵, -OCOR⁵, phenyl, phenyl substituted with W, -C≡CCO₂R⁵, -CH=CHR⁵ or -C≡CR⁵;
each R⁵ is, independently, (C₁-C₄) straight or branched alkyl, phenyl or benzyl, wherein said phenyl and the phenyl moiety of said benzyl may optionally be substituted with halo, hydroxy, nitro, cyano, amino, (C₁-C₄) straight or branched alkyl, (C₁-C₄) straight or branched alkoxy, phenyl, benzyl, (C₁-C₄)alkylamino, di[(C₁-C₄)alkyl]amino, or -S(O)ₘ-(C₁-C₄) straight or branched alkyl;
each W is, independently, halo, R⁵, hydroxy, -OR⁵, nitro, amino, -NHR⁵, -NR⁵R⁵, cyano, or -S(O)ₘ-R⁵;
m is 0, 1 or 2;
n is 1 to 7;
p is 0 or 1;
E¹ and E² are selected, independently, from hydrogen, halo, (C₁-C₃)alkyl, hydroxy, (C₁-C₃)alkoxy, nitro, trifluoromethyl, cyano, amino, (C₁-C₃)alkylamino and di[(C₁-C₃)alkyl]amino;
Het' and Het" are selected, independently, from 6 membered heterocyclic rings containing from one to four nitrogen atoms as part of the ring, optionally substituted with one substituent selected from (C₁-C₃)alkyl, halo, hydroxy, (C₁-C₃)alkoxy, amino, (C₁-C₃)alkylamino and di[(C₁-C₃)alkyl]amino;
(c) compounds of the formula III 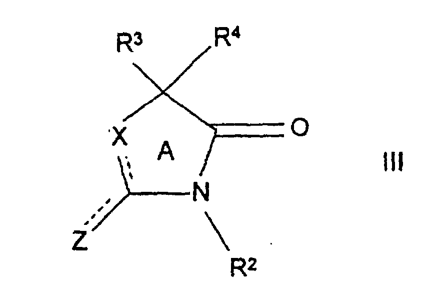 wherein:
both dotted lines represent optional double bonds;
Z is oxygen or sulfur when it is double bonded to ring A and Z is hydroxy, (C₁-C₁₀)alkyl-S-, (C₁-C₁₀)alkyl-SO-, (C₁-C₁₀)alkyl-SO₂-, adamant-2-yl-S-, naphthyl-S-, benzyl-S-, phenyl-C(=O)CH₂-S-, (C₁-C₆)alkyl-O-C(=O)-CH₂-S- or (H,H) (i.e., Z represents two hydrogen atoms, each of which is single bonded to the same carbon of ring A) when Z is single bonded to ring A, and wherein said naphthyl and phenyl and the phenyl moiety of said benzyl may optionally be substituted with from one to three substituents independently selected from (C₁-C₆)alkyl optionally substituted with from one to three fluorine atoms, (C₁-C₆)alkoxy optionally substituted with from one to three fluorine atoms, halo (e.g., chloro, fluoro, bromo or iodo), amino, (C₁-C₆)alkylamino, [di-(C₁-C₆)alkyl]amino, cyano, nitro, (C₁-C₆)alkyl-SOₙ- wherein n is zero, one or two, -COOH, -COO(C₁-C₆)alkyl and -C(O)NH(C₁-C₆)alkyl;
X is NR¹ or CHR¹;
R¹ is hydrogen, (C₁-C₆)alkyl or (C₁-C₆)alkylphenyl when ring A is saturated (i.e., when ring A contains no double bonds) and R¹ is absent when ring A contains a double bond;
R² is selected from naphthyl, phenyl, (C₁-C₆)alkylphenyl, 1-adamantyl, 2-adamantyl, (C₁-C₈) straight or branched alkyl, (C₃-C₁₀) cycloalkyl and (C₈-C₃₀)bicyclic or tricyclic alkyl; wherein said (C₃-C₁₀)cycloalkyl and said (C₈-C₃₀)bicyclic or tricyclic alkyl may optionally be substituted with a hydroxy group; and wherein said adamantyl groups may optionally be substituted with from one to three substituents independently selected from (C₁-C₆)alkyl, halo and hydroxy; and
R³ and R⁴ are independently selected from benzyl, wherein the phenyl moiety of said benzyl may optionally be substituted with an amino or nitro group; hydrogen, phenyl, (N≡C)-(C₁-C₆)alkyl, (C₁-C₆)alkyl-O-C(=O)-(C₁-C₆)alkyl and Het-CH₂, wherein Het is selected from 2-, 3- or 4-pyridinyl, furyl, tetrahydrofuryl, pyrimidyl, pyrazinyl, pyrazolyl, isoxazolyl, thiophenyl and triazolyl;
with the proviso that (a) no more than one of the two dotted lines can represent a double bond in any one compound, (b) when Z is (H, H), X is CH₂, (c) when Z is oxygen or (H, H) and X is CHR¹, R¹ must be hydrogen, (d) when Z is sulfur and X is NR¹, R¹ must be hydrogen, and (e) one of R³ and R⁴ must be Het-CH₂; and
(d) the compound IV 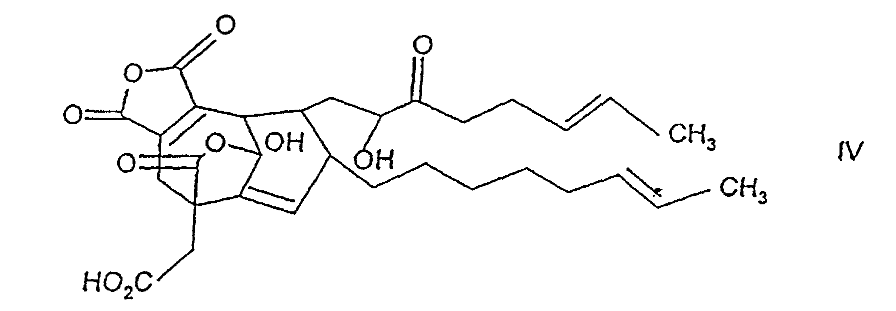 and the pharmaceutically acceptable salts of the foregoing compounds.

2. A pharmaceutical composition according to claim 1, wherein the HMG CoA reductase inhibitor is selected from the group consisting of atorvastatin, pravastatin, lovastatin, compactin fluvastatin and simvastatin, and the pharmaceutically acceptable salts of the foregoing compounds.

3. A composition according to claim 1 or 2 for use as a medicament.

4. The use of FTase inhibitor as defined in claim I and an HMG CoA reductase inhibitor in the preparation of a medicament for the treatment of cancer or a benign proliferative disorder in a mammal.

5. A use according to claim 4 wherein the HMG CoA reductase inhibitor is as defined in claim 2.

6. A pharmaceutical composition for inhibiting the abnormal growth of cells in a mammal, comprising an FTasc inhibitor, an HMG CoA reductase inhibitor and a pharmaceutically acceptable carrier, wherein the FTase inhibitor and the HMG CoA reductase inhibitor are present in amounts that render the composition effective in the inhibition of growth of abnormal cells and wherein the FTase inhibitor is selected from:
(a) compounds of the formula I 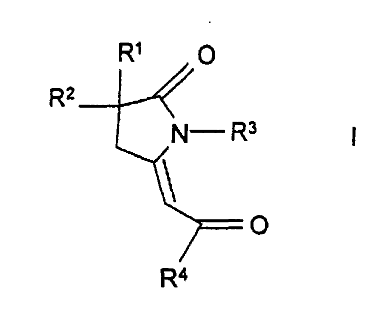 wherein:
R¹ and R² are independently selected from the group consisting of -(CH₂)ₚ(5-10 membered heterocycles), -(CH₂)ₚ(C₆-C₁₀ aryl), allyl, propargyl and C₁-C₆ alkyl wherein p is 0 to 3, said alkyl and the alkyl moieties of said R¹ and R² groups are optionally substituted by 1 to 3 R⁹ substituents, and the aryl and heterocyclic moieties of said R¹ and R² groups are optionally substituted by 1 to 3 substituents independently selected from halo and R⁹;
R³ is -(CH₂)ₘ(1- or 2-adamantyl), -(CH₂)ₘ(C₃-C₁₀ cycloalkyl), -(CH₂)ₘ(C₆-C₁₀ aryl), C₁-C₁₀ alkyl, 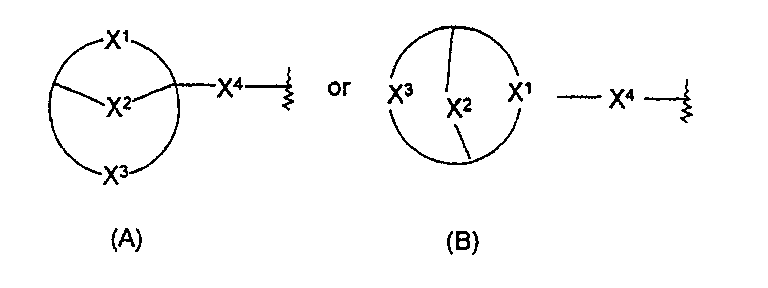
wherein m is 0 to 6, and said cycloalkyl and alkyl optionally contain 1 or 2 double or triple bonds;
X¹, X², and X³ are each independently C₁-C₇ alkylene optionally containing 1 or 2 double or triple bonds, X⁴ is a bond or C₁-C₇ alkylene optionally containing 1 or 2 double or triple bonds, and, in formula (B), the X⁴ moiety is attached to the X¹ moiety at any available carbon in the X¹ moiety;
R⁴ is C₆-C₁₀ aryl, 5-10 membered heterocyclyl or C₁-C₆ alkyl wherein each of said R⁴ groups is optionally substituted by 1 to 3 R⁵ substituents;
each R⁵ is independently selected from the group consisting of halo, nitro, cyano, phenyl, -C(O)OR⁶, -SO₂NR⁶R⁷, -NR⁶R⁸, -C(O)R⁶, -OR⁶, -C(O)NR⁶R⁸, -OC(O)NR⁶R⁸, -NR⁸C(O)NR⁸R⁶, -NR⁸C(O)R⁶, -NR⁸C(O)O(C₁-C₄ alkyl), -C(NR⁸)NR⁸R⁶, -C(NCN)NR⁸R⁶, -C(NCN)S(C₁-C₄ alkyl), -NR⁸C(NCN)S(C₁-C₄ alkyl), -NR⁸C(NCN)NR⁸R⁶, -NR⁸SO₂(C₁-C₄ alkyl), -S(O)ₙ(C₁-C₄ alkyl) wherein n is 0 to 2, -NR⁸C(O)C(O)NR⁸R⁶, -NR⁸C(O)C(O)R⁸, thiazolyl, imidazolyl, oxazolyl, pyrazolyl, triazolyl, tetrazolyl, and C₁-C₄ alkyl optionally substituted by 1 to 3 fluoro substituents;
each R⁶ and R⁷ is independently hydrogen or C₁-C₄ alkyl;
each R⁸ is independently R⁶ or -OR⁶; and,
each R⁹ is independently selected from cyano, R⁶, -OR⁶, -OC(O)R⁶, -C(O)OR⁶, -C(O)NR⁶R⁷, -NR⁶R⁷, -NR⁶R⁸, -SO₂NR⁶R⁷, and C₁-C₄ alkyl substituted by hydroxy;
(b) compounds of the formula IIA or IIB 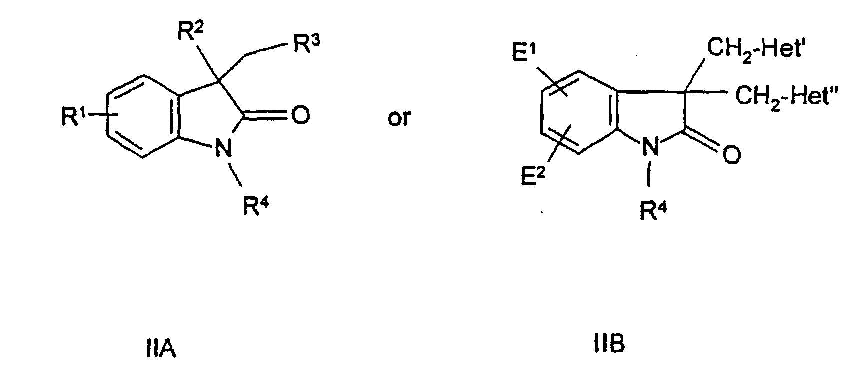 wherein:
R¹ is hydrogen, halo (e.g., chloro, fluoro, bromo or iodo), cyano, hydroxy, nitro, trifluoromethyl, -NHR⁵, -NR⁵R⁵, R⁵, -OR⁵ or -S(O)ₘ-R⁵;
R² is -(CH₂)ₙ-Y or -OCOR⁵;
R³ is 4-, 3-, or 2-pyridyl, pyrimidyl, pyrazinyl, 2-fluoro-4-pyridyl or 3-fluoro-4-pyridyl;
R⁴ is 1-adamantyl or 2-adamantyl;
Y is hydrogen, hydroxy, amino, cyano, -NHR⁵, -NR⁵R⁵, -NHCOR⁵, -NHCO₂R⁵, halo, OR⁵, -S(O)ₘR⁵, -CO₂H, -CO₂R⁵, -CONR⁵R⁵, -CONHR⁵, -CONH₂, -COR⁵, -CH=CHCO₂R⁵, -OCOR⁵, phenyl, phenyl substituted with W, -C≡CCO₂R⁵, -CH=CHR⁵ or -C≡CR⁵;
each R⁵ is, independently, (C₁-C₄) straight or branched alkyl, phenyl or benzyl, wherein said phenyl and the phenyl moiety of said benzyl may optionally be substituted with halo, hydroxy, nitro, cyano, amino, (C₁-C₄) straight or branched alkyl, (C₁-C₄) straight or branched alkoxy, phenyl, benzyl, (C₁-C₄)alkylamino, di[(C₁-C₄)alkyl]amino, or -S(O)ₘ-(C₁-C₄) straight or branched alkyl;
each W is, independently, halo, R⁵, hydroxy, -OR⁵, nitro, amino, -NHR⁵, -NR⁵R⁵, cyano, or -S(O)ₘ-R⁵;
m is 0, 1 or 2;
n is 1 to 7;
p is 0 or 1;
E¹ and E² are selected, independently, from hydrogen, halo, (C₁-C₃)alkyl, hydroxy, (C₁-C₃)alkoxy, nitro, trifluoromethyl, cyano, amino, (C₁-C₃)alkylamino and di[(C₁-C₃)alkyl]amino;
Het' and Het" are selected, independently, from 6 membered heterocyclic rings containing from one to four nitrogen atoms as part of the ring, optionally substituted with one substituent selected from (C₁-C₃)alkyl, halo, hydroxy, (C₁-C₃)alkoxy, amino, (C₁-C₃)alkylamino and di[(C₁-C₃)alkyl]amino;
(c) compounds of the formula III 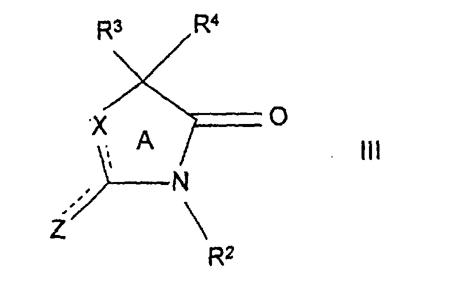 wherein:
both dotted lines represent optional double bonds;
Z is oxygen or sulfur when it is double bonded to ring A and Z is hydroxy, (C₁-C₁₀)alkyl-S-, (C₁-C₁₀)alkyl-SO-, (C₁-C₁₀)alkyl-SO₂-, adamant-2-yl-S-, naphthyl-S-, benzyl-S-, phenyl-C(=O)CH₂-S-, (C₁-C₆)alkyl-O-C(=O)-CH₂-S- or (H,H) (i.e., Z represents two hydrogen atoms, each of which is single bonded to the same carbon of ring A) when Z is single bonded to ring A, and wherein said naphthyl and phenyl and the phenyl moiety of said benzyl may optionally be substituted with from one to three substituents independently selected from (C₁-C₆)alkyl optionally substituted with from one to three fluorine atoms, (C₁-C₆)alkoxy optionally substituted with from one to three fluorine atoms, halo (e.g., chloro, fluoro, bromo or iodo), amino, (C₁-C₆)alkylamino, [di-(C₁-C₆)alkyl]amino, cyano, nitro, (C₁-C₆)alkyl-SOₙ- wherein n is zero, one or two, -COOH, -COO(C₁-C₆)alkyl and -C(O)NH(C₁-C₆)alkyl;
X is NR¹ or CHR¹;
R¹ is hydrogen, (C₁-C₆)alkyl or (C₁-C₆)alkylphenyl when ring A is saturated (i.e., when ring A contains no double bonds) and R¹ is absent when ring A contains a double bond;
R² is selected from naphthyl, phenyl, (C₁-C₆)alkylphenyl, 1-adamantyl, 2-adamantyl, (C₁-C₈) straight or branched alkyl, (C₃-C₁₀) cycloalkyl and (C₈-C₃₀)bicyclic or tricyclic alkyl; wherein said (C₃-C₁₀)cycloalkyl and said (C₈-C₃₀)bicyclic or tricyclic alkyl may optionally be substituted with a hydroxy group; and wherein said adamantyl groups may optionally be substituted with from one to three substituents independently selected from (C₁-C₆)alkyl, halo and hydroxy; and
R³ and R⁴ are independently selected from benzyl, wherein the phenyl moiety of said benzyl may optionally be substituted with an amino or nitro group; hydrogen, phenyl, (N≡C)-(C₁-C₆)alkyl, (C₁-C₆)alkyl-O-C(=O)-(C₁-C₆)alkyl and Het-CH₂, wherein Het is selected from 2-, 3- or 4-pyridinyl, furyl, tetrahydrofuryl, pyrimidyl, pyrazinyl, pyrazolyl, isoxazol thiophenyl and triazolyl;
with the proviso that (a) no more than one of the two dotted lines can represent a double bond in any one compound, (b) when Z is (H, H), X is CH₂, (c) when Z is oxygen or (H, H) and X is CHR¹, R¹ must be hydrogen, (d) when Z is sulfur and X is NR¹, R¹ must be hydrogen, and (e) one of R³ and R⁴ must be Het-CH₂; and
(d) the compound IV 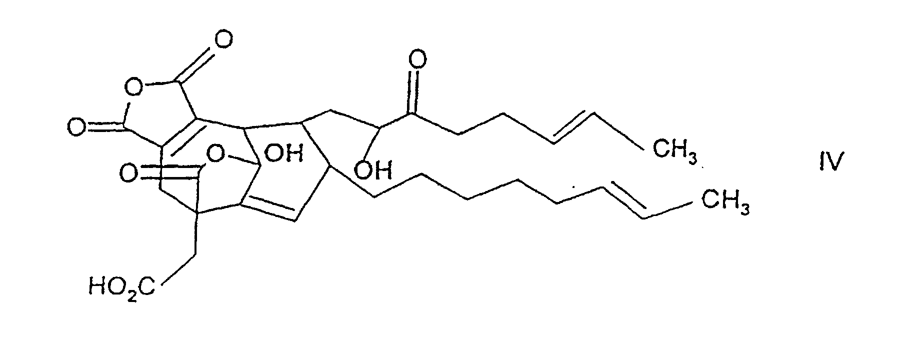 and the pharmaceutically acceptable salts of the foregoing compounds.

7. A pharmaceutical composition according to claim 5, wherein the HMG CoA reductase inhibitor is selected from the group consisting of atorvastatin, pravastatin, lovastatin, compactin fluvastatin and simvastatin, and the pharmaceutically acceptable salts of the foregoing compounds.

8. A composition according to claim 5 or 6 for use as a medicament.

9. The use of an FTase inhibitor as defined in claim 5 and an HMG CoA reductase inhibitor in the preparation of a medicament of the treatment of a condition in the mammal for which the inhibition of abnormal growth of cells is indicated.

10. A use according to claim 9 wherein the HMG CoA reductase inhibitor is as defined in claim 7.

## Patentansprüche

1. Pharmazeutische Zusammensetzung zur Behandlung von Krebs oder einer benignen proliferativen Störung bei einem Säugetier, enthaltend einen FTase-Inhibitor, einen HMG-CoA-Reduktaseinhibitor und einen pharmazeutisch annehmbaren Träger, wobei der FTase-Inhibitor und der HMG-CoA-Reduktaseinhibitor in solchen Mengen vorhanden sind, die die Zusammensetzung zur Behandlung von Krebs oder einer benignen proliferativen Störung wirksam machen und wobei der FTase-Inhibitor ausgewählt ist aus:
a) Verbindungen der Formel I 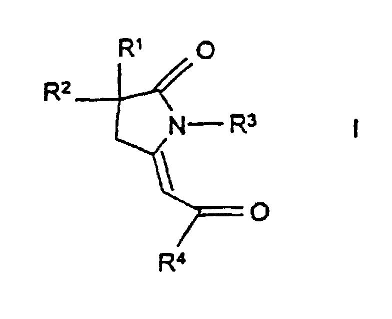 worin R¹ und R² unabhängig ausgewählt sind aus der Gruppe bestehend aus -(CH₂)ₚ(5 - 10-gliedrigen Heterocyclen), -(CH₂)ₚ(C₆-C₁₀-Aryl), Allyl-, Propargylund C₁-C₆-Alkylgruppen, wobei p 0 bis 3 ist, wobei die Alkylgruppen und Alkylanteile der Gruppen R¹ und R² gegebenenfalls mit 1 bis 3 Substituenten R⁹ substituiert sind und die Arylgruppen und heterocyclischen Anteile der Gruppen R¹ und R² gegebenenfalls mit 1 bis 3 Substituenten substituiert sind, die unabhängig ausgewählt sind aus Halogen und R⁹; R³ -(CH₂)ₘ(1- oder 2-Adamantyl), -(CH₂)ₘ(C₃-C₁₀-Cycloalkyl), -(CH₂)ₘ(C₆-C₁₀-Aryl), eine C₁-C₁₀-Alkylgruppe, 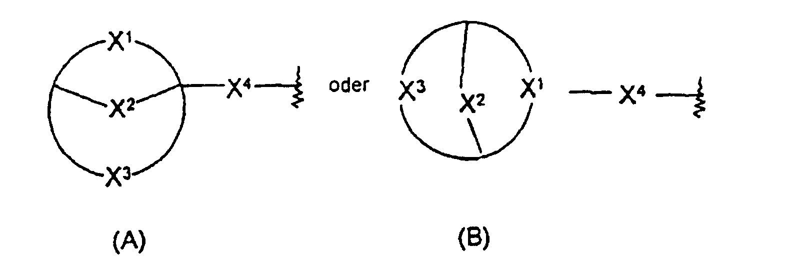 ist, worin m 0 bis 6 ist und die Cycloalkyl- und Alkylgruppen gegebenenfalls 1 oder 2 Doppel- oder Dreifachbindungen enthalten;
X¹, X² und X³ jeweils unabhängig C₁-C₇-Alkylenreste, die gegebenenfalls 1 oder 2 Doppel- oder Dreifachbindungen enthalten, sind, X⁴ eine Bindung oder ein C₁-C₇-Alkylenrest ist, der gegebenenfalls 1 oder 2 Doppel- oder Dreifachbindungen enthält, und in Formel (B) der X⁴-Anteil an den X¹-Anteil an jedem verfügbaren Kohlenstoffatom in dem X¹-Anteil gebunden ist;
R⁴ eine C₆-C₁₀-Aryl-, 5- bis 10-gliedrige Heterocyclyl- oder C₁-C₆-Alkylgruppe ist, worin jede der Gruppen R⁴ gegebenenfalls mit 1 bis 3 Substituenten R⁵ substituiert ist;
jeder Rest R⁵ unabhängig ausgewählt ist aus der Gruppe bestehend aus Halogen, Nitro, Cyano, Phenyl, -C(O)OR⁶, -SO₂NR⁶R⁷, -NR⁶R⁸, -C(O)R⁶, -OR⁶, -C(O)NR⁶R⁸, -OC(O)NR⁶R⁸, -NR⁸C(O)NR⁸R⁶, -NR⁸C(O)R⁶, -NR⁸C(O)O (C₁-C₄-Alkyl), -C(NR⁸)NR⁸R⁶, -C(NCN)NR⁸R⁶, -C(NCN)S(C₁-C₄-Alkyl), -NR⁸C(NCN)S(C₁-C₄-Alkyl), -NR⁸C(NCN)NR⁸R⁶, -NR⁸SO₂(C₁-C₄-Alkyl), -S(O)ₙ(C₁-C₄-Alkyl), worin n 0 bis 2 ist, -NR⁸C(O)C(O)NR⁸R⁶, -NR⁸C(O)C(O)R⁸, Thiazolyl, Imidazolyl, Oxazolyl, Pyrazolyl, Triazolyl, Tetrazolyl und C₁-C₄-Alkylgruppen, die gegebenenfalls mit 1 bis 3 Fluorsubstituenten substituiert sind; jeder Rest R⁶ und R⁷ unabhängig Wasserstoff oder ein C₁-C₄-Alkylrest ist;
jeder Rest R⁸ unabhängig R⁶ oder -OR⁶ ist und
jeder Rest R⁹ unabhängig ausgewählt ist aus Cyano, R⁶, -OR⁶, -OC(O)R⁶, -C(O)OR⁶, -C(O)NR⁶R⁷, NR⁶R⁷, NR⁶R⁸, -SO₂NR⁶R⁷ und C₁-C₄-Alkylresten, die mit Hydroxyresten substituiert sind;
b) Verbindungen der Formel IIA oder IIB 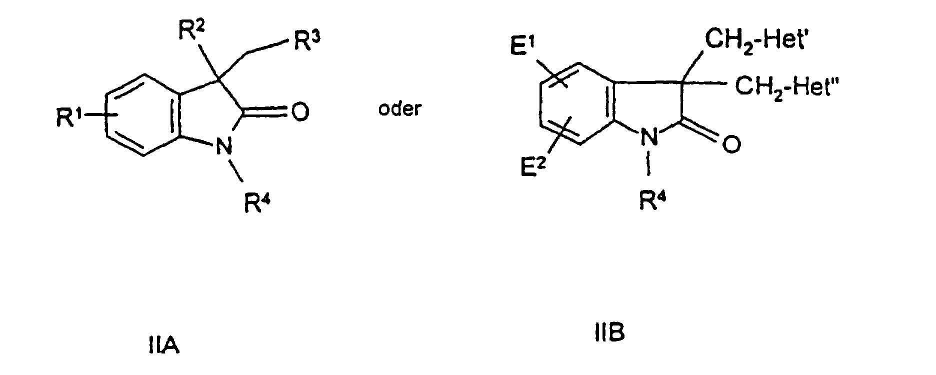 worin
R¹ Wasserstoff, Halogen (z.B. Chlor, Fluor, Brom oder Iod), ein Cyano-, Hydroxy-, Nitro-, Trifluormethylrest, -NHR⁵, -NR⁵R⁵, R⁵, -OR⁵ oder -S(O)ₘ-R⁵ ist;
R² -(CH₂)ₙ-Y oder -OCOR⁵ ist;
R³ eine 4-, 3- oder 2-Pyridyl-, Pyrimidyl-, Pyrazinyl-, 2-Fluor-4-pyridyl oder 3-Fluor-4-pyridylgruppe ist;
R⁴ eine 1-Adamantyl- oder 2-Adamantylgruppe ist;
Y Wasserstoff, ein Hydroxy-, Amino-, Cyanorest, -NHR⁵, -NR⁵R⁵, -NHCOR⁵, -NHCO₂R⁵, Halogen, OR⁵, -S(O)ₘR⁵, -CO₂H, -CO₂R⁵, -CONR⁵R⁵, -CONHR⁵, -CONH₂, -COR⁵, -CH=CHCO₂R⁵, -OCOR⁵, Phenylrest, mit W substituierter Phenylrest, -C≡CCO₂R⁵, -CH=CHR⁵ oder -C≡CR⁵ ist;
jeder Rest R⁵ unabhängig ein geradkettiger oder verzweigter C₁-C₄-Alkyl-, Phenyl- oder Benzylrest ist, wobei der Phenylrest und der Phenylanteil des Benzylrestes gegebenenfalls mit Halogen-, Hydroxy-, Nitro-, Cyano-, Amino-, geradkettigen oder verzweigten (C₁-C₄)-Alkyl-, geradkettigen oder verzweigten (C₁-C₄)-Alkoxy-, Phenyl-, Benzyl-, (C₁-C₄)-Alkylamino-, Di[(C₁-C₄)-alkyl]amino- oder geradkettigen oder verzweigten -S(O)ₘ-(C₁-C₄)-Alkylresten substituiert sein kann;
jeder Rest W unabhängig Halogen, R⁵, Hydroxy, -OR⁵, Nitro, Amino, -NHR⁵, -NR⁵R⁵, Cyano oder -S(O)ₘ-R⁵ ist;
m 0, 1 oder 2 ist;
n 1 bis 7 ist;
p 0 oder 1 ist;
E¹ und E² unabhängig ausgewählt sind aus Wasserstoff, Halogen-, (C₁-C₃)-Alkyl-, Hydroxy-, (C₁-C₃)-Alkoxy-, Nitro-, Trifluormethyl-, Cyano-, Amino-, (C₁-C₃)-Alkylamino- und Di[(C₁-C₃)-alkyl]aminoresten;
Het' und Het'' unabhängig ausgewählt sind aus 6-gliedrigen heterocyclischen Ringen, die 1 bis 4 Stickstoffatome als Teil des Rings enthalten, die gegebenenfalls mit einem Substituenten substituiert sind ausgewählt aus (C₁-C₃)-Alkyl-, Halogen-, Hydroxy-, (C₁-C₃)-Alkoxy-, Amino-, (C₁-C₃)-Alkylamino- und Di[(C₁-C₃)-alkyl]aminoresten;
c) Verbindungen der Formel III 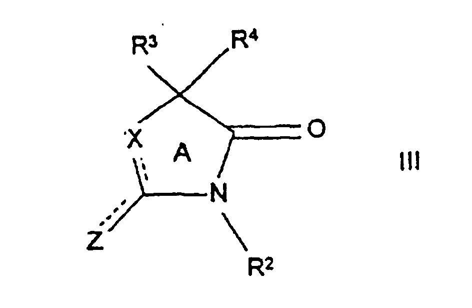 worin
beide gepunktete Linien fakultative Doppelbindungen bedeuten;
Z Sauerstoff oder Schwefel ist, wenn es doppelt an den Ring A gebunden ist, und worin Z ein Hydroxy-, (C₁-C₁₀)-Alkyl-S-, (C₁-C₁₀)-Alkyl-SO-, (C₁-C₁₀)-Alkyl-SO₂-, Adamant-2-yl-S-, Naphthyl-S-, Benzyl-S-, Phenyl-C(=O)CH₂-S-, (C₁-C₆)-Alkyl-O-C(=O)-CH₂-S-Rest oder (H,H) ist (d.h. Z bedeutet zwei Wasserstoffatome, von denen jedes einfach an das gleiche Kohlenstoffatom des Rings A gebunden ist), wenn Z einfach an den Ring A gebunden ist, und wobei die Naphthyl- und Phenylgruppen und der Phenylanteil des Benzylrests gegebenenfalls mit 1 bis 3 Substituenten substituiert sein können, die unabhängig ausgewählt sind aus gegebenenfalls mit 1 bis 3 Fluoratomen substituierten (C₁-C₆)-Alkyl-, gegebenenfalls mit 1 bis 3 Fluoratomen substituierten (C₁-C₆)-Alkoxy-, Halogen- (z.B. Chlor, Fluor, Brom oder Iod), Amino-, (C₁-C₆)-Alkylamino-, [Di(C₁-C₆)-alkyl]amino-, Cyano-, Nitro-, (C₁-C₆)-Alkyl-SOₙ-Resten, worin n 0, 1 oder 2 ist, -COOH, -COO(C₁-C₆)-Alkyl und -C(O)NH(C₁-C₆)-Alkyl;
X NR¹ oder CHR¹ ist;
R¹ Wasserstoff, ein (C₁-C₆)-Alkyl- oder (C₁-C₆)-Alkylphenylrest ist, wenn Ring A gesättigt ist (d.h. wenn Ring A keine Doppelbindungen enthält) und R¹ nicht vorhanden ist, wenn Ring A eine Doppelbindung enthält;
R² ausgewählt ist aus Naphthyl-, Phenyl-, (C₁-C₆)-Alkylphenyl-, 1-Adamantyl-, 2-Adamantyl-, geradkettigen oder verzweigten (C₁-C₈)-Alkyl-, (C₃-C₁₀)-Cycloalkyl- und bicyclischen oder tricyclischen (C₈-C₃₀)-Alkylgruppen, wobei die (C₃-C₁₀)-Cycloalkylgruppen und die bicyclischen oder tricyclischen (C₈-C₃₀)-Alkylgruppen gegebenenfalls mit einer Hydroxygruppe substituiert sein können; und wobei die Adamantylgruppen gegebenenfalls mit 1 bis 3 Substituenten substituiert sein können, die unabhängig ausgewählt sind aus (C₁-C₆)-Alkyl-, Halogen- und Hydroxyresten; und
R³ und R⁴ unabhängig ausgewählt sind aus Benzylgruppen, wobei der Phenylanteil der Benzylgruppe gegebenenfalls mit einer Amino- oder Nitrogruppe substituiert sein kann; Wasserstoff, Phenyl, (N≡C)-(C₁-C₆)-Alkyl, (C₁-C₆)-Alkyl-O-C(=O)-(C₁-C₆)-alkyl und Het-CH₂, wobei Het ausgewählt ist aus 2-, 3- oder 4-Pyridinyl-, Furyl-, Tetrahydrofuryl-, Pyrimidyl-, Pyrazinyl-, Pyrazolyl-, Isoxazolyl-, Thiophenyl- und Triazolylgruppen;
mit dem Vorbehalt, dass (a) nicht mehr als eine der zwei gepunkteten Linien eine Doppelbindung in jeder Verbindung bedeuten kann, (b) wenn Z (H, H) ist, X CH₂ ist, (c) wenn Z Sauerstoff oder (H, H) ist und X CHR¹ ist, R¹ Wasserstoff sein muss, (d) wenn Z Schwefel ist und X NR¹ ist, R¹ Wasserstoff sein muss und (e) einer der Reste R³ und R⁴ Het-CH₂ sein muss und
d) der Verbindung IV 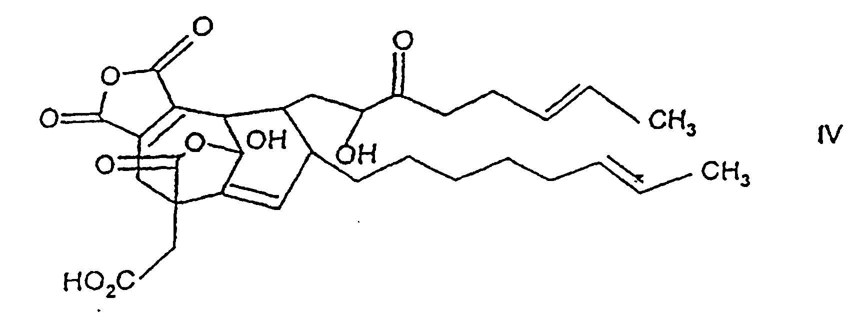 und den pharmazeutisch annehmbaren Salzen der vorhergehenden Verbindungen.

2. Pharmazeutische Zusammensetzung nach Anspruch 1, wobei der HMG-CoA-Reduktaseinhibitor ausgewählt ist aus der Gruppe bestehend aus Atorvastatin, Pravastatin, Lovastatin, Compactin, Fluvastatin und Simvastatin, und den pharmazeutisch annehmbaren Salzen der vorhergehenden Verbindungen.

3. Zusammensetzung nach Anspruch 1 oder Anspruch 2 zur Verwendung als Arzneimittel.

4. Verwendung eines FTase-Inhibitors wie in Anspruch 1 definiert und eines HMG-CoA-Reduktaseinhibitors zur Herstellung eines Arzneimittels zur Behandlung von Krebs oder einer benignen proliferativen Störung bei einem Säugetier.

5. Verwendung nach Anspruch 4, wobei der HMG-CoA-Reduktaseinhibitor wie in Anspruch 2 definiert ist.

6. Pharmazeutische Zusammensetzung zur Hemmung des anormalen Wachstums von Zellen bei einem Säugetier enthaltend einen FTase-Inhibitor, einen HMG-CoA-Reduktaseinhibitor und einen pharmazeutisch annehmbaren Träger, wobei der FTase-Inhibitor und der HMG-CoA-Reduktaseinhibitor in Mengen vorhanden sind, die die Zusammensetzung zur Hemmung des Wachstums von anormalen Zellen wirksam machen und wobei der FTase-Inhibitor ausgewählt ist aus:
a) Verbindungen der Formel I 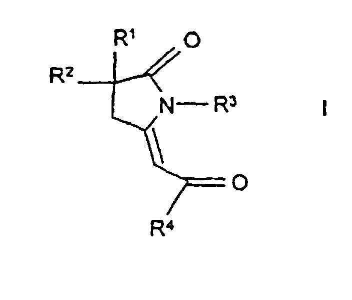 worin R¹ und R² unabhängig ausgewählt sind aus der Gruppe bestehend aus -(CH₂)ₚ(5 - 10-gliedrigen Heterocyclen), - (CH₂)ₚ(C₆-C₁₀-Aryl), Allyl-, Propargylund C₁-C₆-Alkylgruppen, wobei p 0 bis 3 ist, wobei die Alkylgruppen und Alkylanteile der Gruppen R¹ und R² gegebenenfalls mit 1 bis 3 Substituenten R⁹ substituiert sind und die Arylgruppen und heterocyclischen Anteile der Gruppen R¹ und R² gegebenenfalls mit 1 bis 3 Substituenten substituiert sind, die unabhängig ausgewählt sind aus Halogen und R⁹; R³ -(CH₂)ₘ(1- oder 2-Adamantyl), -(CH₂)ₘ(C₃-C₁₀-Cycloalkyl), -(CH₂)ₘ(C₆-C₁₀-Aryl), eine C₁-C₁₀-Alkylgruppe, 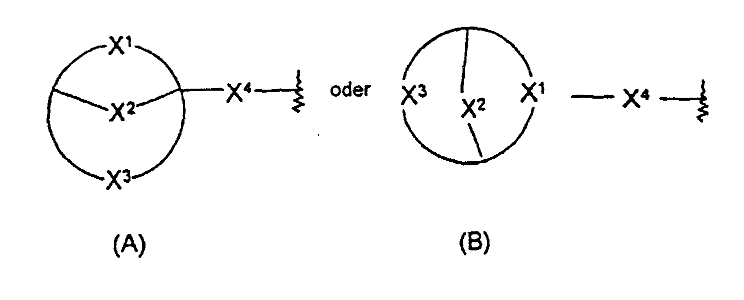 ist, worin m 0 bis 6 ist und die Cycloalkyl- und Alkylgruppen gegebenenfalls 1 oder 2 Doppel- oder Dreifachbindungen enthalten;
X¹, X² und X³ jeweils unabhängig C₁-C₇-Alkylenreste, die gegebenenfalls 1 oder 2 Doppel- oder Dreifachbindungen enthalten, sind, X⁴ eine Bindung oder ein C₁-C₇-Alkylenrest ist, der gegebenenfalls 1 oder 2 Doppel- oder Dreifachbindungen enthält, und in Formel (B) der X⁴-Anteil an den X¹-Anteil an jedem verfügbaren Kohlenstoffatom in dem X¹-Anteil gebunden ist;
R⁴ eine C₆-C₁₀-Aryl-, 5- bis 10-gliedrige Heterocyclyl- oder C₁-C₆-Alkylgruppe ist, worin jede der Gruppen R⁴ gegebenenfalls mit 1 bis 3 Substituenten R⁵ substituiert ist;
jeder Rest R⁵ unabhängig ausgewählt ist aus der Gruppe bestehend aus Halogen, Nitro, Cyano, Phenyl, -C(O)OR⁶, -SO₂NR⁶R⁷, -NR⁶R⁸, -C(O)R⁶, -OR⁶, -C(O)NR⁶R⁸, -OC(O)NR⁶R⁸, -NR⁸C(O)NR⁸R⁶, -NR⁸C(O)R⁶, -NR⁸C(O)O(C₁-C₄-Alkyl), -C(NR⁸)NR⁸R⁶, -C(NCN)NR⁸R⁶, -C(NCN)S(C₁-C₄-Alkyl), -NR⁸C(NCN)S(C₁-C₄-Alkyl), -NR⁸C(NCN)NR⁸R⁶, -NR⁸SO₂(C₁-C₄-Alkyl), -S(O)ₙ(C₁-C₄-Alkyl), worin n 0 bis 2 ist, -NR⁸C(O)C(O)NR⁸R⁶, -NR⁸C(O)C(O)R⁸, Thiazolyl, Imidazolyl, Oxazolyl, Pyrazolyl, Triazolyl, Tetrazolyl und C₁-C₄-Alkylgruppen, die gegebenenfalls mit 1 bis 3 Fluorsubstituenten substituiert sind;
jeder Rest R⁶ und R⁷ unabhängig Wasserstoff oder ein C₁-C₄-Alkylrest ist;
jeder Rest R⁸ unabhängig R⁶ oder -OR⁶ ist und
jeder Rest R⁹ unabhängig ausgewählt ist aus Cyano, R⁶, -OR⁶, -OC(O)R⁶, -C(O)OR⁶, -C(O)NR⁶R⁷, NR⁶R⁷, NR⁶R⁸, -SO₂NR⁶R⁷ und C₁-C₄-Alkylresten, die mit Hydroxyresten substituiert sind;
b) Verbindungen der Formel IIA oder IIB 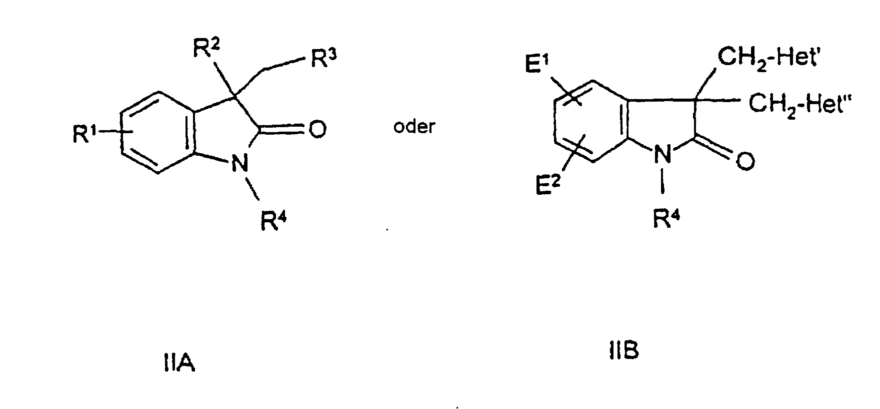 worin
R¹ Wasserstoff, Halogen (z.B. Chlor, Fluor, Brom oder Iod), ein Cyano-, Hydroxy-, Nitro-, Trifluormethylrest, -NHR⁵, -NR⁵R⁵, R⁵, -OR⁵ oder -S(O)ₘ-R⁵ ist;
R²-(CH₂)ₙ-Y oder -OCOR⁵ ist;
R³ eine 4-, 3- oder 2-Pyridyl-, Pyrimidyl-, Pyrazinyl-, 2-Fluor-4-pyridyl oder 3-Fluor-4-pyridylgruppe ist;
R⁴ eine 1-Adamantyl- oder 2-Adamantylgruppe ist;
Y Wasserstoff, ein Hydroxy-, Amino-, Cyanorest, -NHR⁵, -NR⁵R⁵, -NHCOR⁵, -NHCO₂R⁵, Halogen, OR⁵, -S(O)ₘR⁵, -CO₂H, -CO₂R⁵, -CONR⁵R⁵, -CONHR⁵, -CONH₂, -COR⁵, -CH=CHCO₂R⁵, -OCOR⁵, Phenylrest, mit W substituierter Phenylrest, -C≡CCO₂R⁵, -CH=CHR⁵ oder -C≡CR⁵ ist;
jeder Rest R⁵ unabhängig ein geradkettiger oder verzweigter C₁-C₄-Alkyl-, Phenyl- oder Benzylrest ist, wobei der Phenylrest und der Phenylanteil des Benzylrestes gegebenenfalls mit Halogen-, Hydroxy-, Nitro-, Cyano-, Amino-, geradkettigen oder verzweigten (C₁-C₄)-Alkyl-, geradkettigen oder verzweigten (C₁-C₄)-Alkoxy-, Phenyl-, Benzyl-, (C₁-C₄)-Alkylamino-, Di[(C₁-C₄)-alkyl]amino- oder geradkettigen oder verzweigten -S(O)ₘ-(C₁-C₄)-Alkylresten substituiert sein kann;
jeder Rest W unabhängig Halogen, R⁵, Hydroxy, -OR⁵_{,} Nitro, Amino, -NHR⁵, -NR⁵R⁵, Cyano oder -S(O)ₘ-R⁵ ist; m 0, 1 oder 2 ist;
n 1 bis 7 ist;
p 0 oder 1 ist;
E¹ und E² unabhängig ausgewählt sind aus Wasserstoff, Halogen-, (C₁-C₃)-Alkyl-, Hydroxy-, (C₁-C₃)-Alkoxy-, Nitro-, Trifluormethyl-, Cyano-, Amino-, (C₁-C₃)-Alkylamino- und Di[(C₁-C₃)-alkyl]aminoresten;
Het' und Het" unabhängig ausgewählt sind aus 6-gliedrigen heterocyclischen Ringen, die 1 bis 4 Stickstoffatome als Teil des Rings enthalten, die gegebenenfalls mit einem Substituenten substituiert sind ausgewählt aus (C₁-C₃)-Alkyl-, Halogen-, Hydroxy-, (C₁-C₃)-Alkoxy-, Amino-, (C₁-C₃)-Alkylamino- und Di [(C₁-C₃)-alkyl]aminoresten;
c) Verbindungen der Formel III 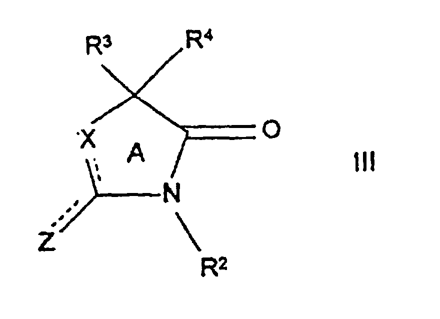 worin
beide gepunktete Linien fakultative Doppelbindungen bedeuten;
Z Sauerstoff oder Schwefel ist, wenn es doppelt an den Ring A gebunden ist, und worin Z ein Hydroxy-, (C₁-C₁₀)-Alkyl-S-, (C₁-C₁₀)-Alkyl-SO-, (C₁-C₁₀)-Alkyl-SO₂-, Adamant-2-yl-S-, Naphthyl-S-, Benzyl-S-, Phenyl-C(=O)CH₂-S-, (C₁-C₆)-Alkyl-O-C(=O)-CH₂-S-Rest oder (H,H) ist (d.h. Z bedeutet zwei Wasserstoffatome, von denen jedes einfach an das gleiche Kohlenstoffatom des Rings A gebunden ist), wenn Z einfach an den Ring A gebunden ist, und wobei die Naphthyl- und Phenylgruppen und der Phenylanteil des Benzylrests gegebenenfalls mit 1 bis 3 Substituenten substituiert sein können, die unabhängig ausgewählt sind aus gegebenenfalls mit 1 bis 3 Fluoratomen substituierten (C₁-C₆)-Alkyl-, gegebenenfalls mit 1 bis 3 Fluoratomen substituierten (C₁-C₆)-Alkoxy-, Halogen- (z.B. Chlor, Fluor, Brom oder Iod), Amino-, (C₁-C₆)-Alkylamino-, [Di (C₁-C₆)-alkyl]amino-, Cyano-, Nitro-, (C₁-C₆)-Alkyl-SOₙ-Resten, worin n 0, 1 oder 2 ist, -COOH, -COO(C₁-C₆) -Alkyl und -C(O)NH(C₁-C₆)-Alkyl;
X NR¹ oder CHR¹ ist;
R¹ Wasserstoff, ein (C₁-C₆)-Alkyl- oder (C₁-C₆)-Alkylphenylrest ist, wenn Ring A gesättigt ist (d.h. wenn Ring A keine Doppelbindungen enthält) und R¹ nicht vorhanden ist, wenn Ring A eine Doppelbindung enthält;
R² ausgewählt ist aus Naphthyl-, Phenyl-, (C₁-C₆)-Alkylphenyl-, 1-Adamantyl-, 2-Adamantyl-, geradkettigen oder verzweigten (C₁-C₈)-Alkyl-, (C₃-C₁₀)-Cycloalkyl- und bicyclischen oder tricyclischen (C₈-C₃₀)-Alkylgruppen, wobei die (C₃-C₁₀)-Cycloalkylgruppen und die bicyclischen oder tricyclischen (C₈-C₃₀)-Alkylgruppen gegebenenfalls mit einer Hydroxygruppe substituiert sein können; und wobei die Adamantylgruppen gegebenenfalls mit 1 bis 3 Substituenten substituiert sein können, die unabhängig ausgewählt sind aus (C₁-C₆)-Alkyl-, Halogen- und Hydroxyresten; und
R³ und R⁴ unabhängig ausgewählt sind aus Benzylgruppen, wobei der Phenylanteil der Benzylgruppe gegebenenfalls mit einer Amino- oder Nitrogruppe substituiert sein kann; Wasserstoff, Phenyl, (N≡C)-(C₁-C₆)-Alkyl, (C₁-C₆)-Alkyl-O-C(=O)-(C₁-C₆)-alkyl und Het-CH₂, wobei Het ausgewählt ist aus 2-, 3- oder 4-Pyridinyl-, Furyl-, Tetrahydrofuryl-, Pyrimidyl-, Pyrazinyl-, Pyrazolyl-, Isoxazolyl-, Thiophenyl- und Triazolylgruppen;
mit dem Vorbehalt, dass (a) nicht mehr als eine der zwei gepunkteten Linien eine Doppelbindung in jeder Verbindung bedeuten kann, (b) wenn Z (H, H) ist, X CH₂ ist, (c) wenn Z Sauerstoff oder (H, H) ist und X CHR¹ ist, R¹ Wasserstoff sein muss, (d) wenn Z Schwefel ist und X NR¹ ist, R¹ Wasserstoff sein muss und (e) einer der Reste R³ und R⁴ Het-CH₂ sein muss und
d) der Verbindung IV 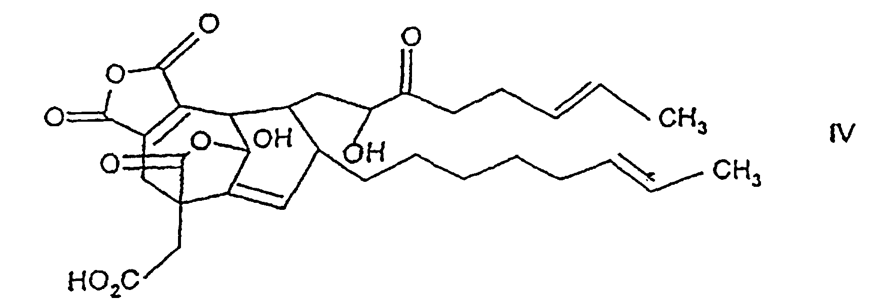 und den pharmazeutisch annehmbaren Salzen der vorhergehenden Verbindungen.

7. Pharmazeutische Zusammensetzung nach Anspruch 5, wobei der HMG-CoA-Reduktaseinhibitor ausgewählt ist aus der Gruppe bestehend aus Atorvastatin, Pravastatin, Lovastatin, Compactin, Fluvastatin und Simvastatin, und den pharmazeutisch annehmbaren Salzen der vorhergehenden Verbindungen.

8. Zusammensetzung nach Anspruch 5 oder Anspruch 6 zur Verwendung als Arzneimittel.

9. Verwendung eines FTase-Inhibitors, wie in Anspruch 5 definiert, und eines HMG-CoA-Reduktaseinhibitors zur Herstellung eines Arzneimittels zur Behandlung eines Zustandes bei einem Säugetier, bei dem die Hemmung des anormalen Wachstums von Zellen indiziert ist.

10. Verwendung nach Anspruch 9, wobei der HMG-CoA-Reduktaseinhibitor wie in Anspruch 7 definiert ist.

## Revendications

1. Composition pharmaceutique pour le traitement du cancer ou d'un trouble prolifératif bénin chez un mammifère, comprenant un inhibiteur de FTase, un inhibiteur de HMG-CoA-réductase et un support pharmaceutiquement acceptable, dans laquelle l'inhibiteur de FTase et l'inhibiteur de HMG-CoA-réductase sont présents en des quantités qui rendent la composition efficace dans le traitement du cancer ou d'un trouble prolifératif bénin, et dans laquelle l'inhibiteur de FTase est choisi entre :
(a) des composés de formule I 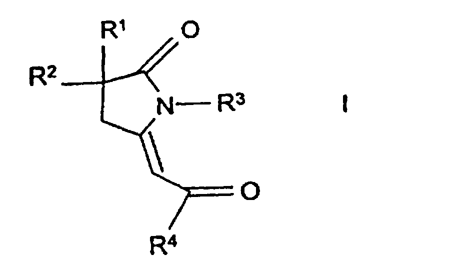 dans laquelle :
R¹ et R² sont choisis, indépendamment, dans le groupe consistant en des groupes -(CH₂)ₚ(hétérocycles penta- à décagonaux), -(CH₂)ₚ(aryle en C₆ à C₁₀), allyle, propargyle et alkyle en C₁ à C₆ dans lesquels p a une valeur de 0 à 3, lesdits groupes alkyle et les groupements alkyle desdits groupes R¹ et R² sont facultativement substitués avec 1 à 3 substituants R⁹, et les groupements aryle et hétérocycliques desdits groupes R¹ et R² sont facultativement substitués avec 1 à 3 substituants choisis, indépendamment, entre des substituants halogéno et R⁹ ;
R³ représente un groupe -(CH₂)ₘ(1- ou 2-adamantyle), -(CH₂)ₘ(cycloalkyle en C₃ à C₁₀), -(CH₂)ₘ(aryle en C₆ à C₁₀), alkyle en C₁ à C₁₀, 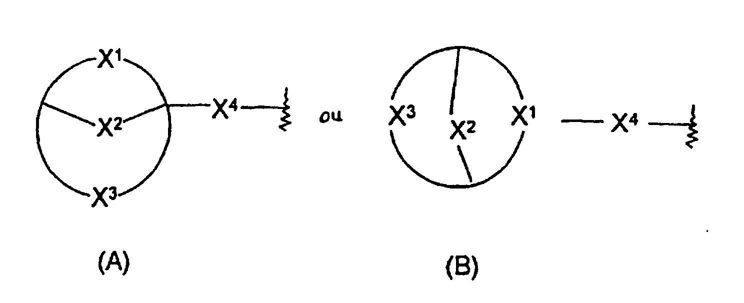 dans lequel m a une valeur de 0 à 6, et lesdits groupes cycloalkyle et alkyle contiennent facultativement 1
ou 2 doubles ou triples liaisons ;
X¹, X² et X³ représentent chacun, indépendamment, un groupe alkylène en C₁ à C₇ contenant facultativement 1 ou 2 doubles ou triples liaisons, X⁴ représente une liaison ou un groupe alkylène en C₁ à C₇ contenant facultativement 1 ou 2 doubles ou triples liaisons et, dans la formule (B), le groupement X⁴ est fixé au groupement R¹ au niveau de n'importe quel atome de carbone disponible dans le groupement X¹ ;
R⁴ représente un groupe aryle en C₆ à C₁₀, hétérocycle penta- à décagonal ou alkyle en C₁ à C₆, chacun desdits groupes R⁴ étant facultativement substitué avec 1 à 3 substituants R⁵ ;
chaque groupe R⁵ est choisi, indépendamment, dans le groupe consistant en des groupes halogéno, nitro, cyano, phényle, -C(O)OR⁶, -SO₂NR⁶R⁷, -NR⁶R⁸, -C(O)R⁶, -OR⁶, -C(O)NR⁶R⁸, -OC(O)NR⁶R⁸, -NR⁸C(O)NR⁸R⁶, -NR⁸C(O)R⁶, -NR⁸C(O)O(alkyle en C₁ à C₄), -C(NR⁸)NR⁸R⁶, -C(NCN)NR⁸R⁶, -C(NCN)S(alkyle en C₁ à C₄), -NR⁸C(NCN)S(alkyle en C₁ à C₄), -NR⁸C(NCN)NR⁸R⁶, -NR⁸SO₂(alkyle en C₁ à C₄), -S(O)ₙ(alkyle en C₁ en C₄) dans lequel n a une valeur de 0 à 2, -NR⁸C(O)C(O)NR⁸R⁶, -NR⁸C(O)C(O)R⁸, thiazolyle, imidazolyle, oxazolyle, pyrazolyle, triazolyle, tétrazolyle et alkyle en C₁ à C₄ facultativement substitué avec 1 à 3 substituants fluoro ;
chacun des groupes R⁶ et R⁷ représente, indépendamment, un atome d'hydrogène ou un groupe alkyle en C₁ à C₄ ;
chaque groupe R⁸ représente, indépendamment, un groupe R⁶ ou -OR⁶ ; et
chaque groupe R⁹ est choisi, indépendamment, entre des groupes cyano, R⁶, -OR⁶, -OC(O)R⁶, -C(O)OR⁶, -C(O)NR⁶R⁷, -NR⁶R⁷, -NR⁶R⁸, -SO₂NR⁶R⁷ et alkyle en C₁ à C₄ substitué avec un substituant hydroxy ;
(b) des composés de formule IIA ou IIB 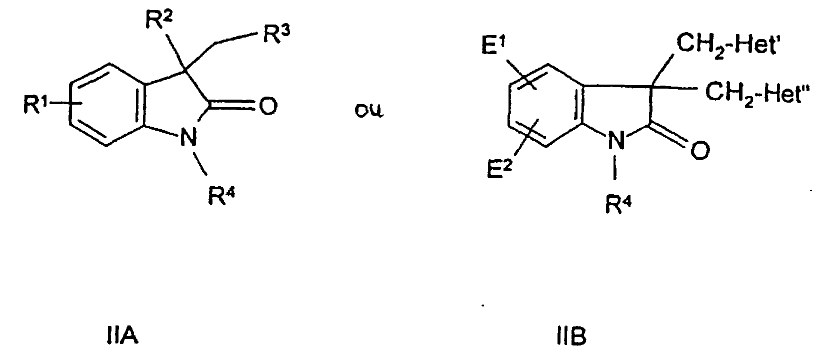 dans laquelle :
R¹ représente un atome d'hydrogène, un groupe halogéno (par exemple chloro, fluoro, bromo ou iodo), cyano, hydroxy, nitro, trifluorométhyle, -NHR⁵, -NR⁵R⁵, R⁵, -OR⁵ ou -S(O)ₘ-R⁵ ;
R² représente un groupe -(CH₂)ₙ-Y ou -OCOR⁵ ;
R³ représente un groupe 4-, 3- ou 2-pyridyle, pyrimidyle, pyrazinyle, 2-fluoro-4-pyridyle ou 3-fluoro-4-pyridyle ;
R⁴ représente un groupe 1-adamantyle ou 2-adamantyle ;
Y représente un atome d'hydrogène, un groupe hydroxy, amino, cyano, -NHR⁵, -NR⁵R⁵, -NHCOR⁵, -NHCO₂R⁵, halogéno, OR⁵, -S(O)ₘR⁵, -CO₂H, -CO₂R⁵, -CONR⁵R⁵, -CONHR⁵, -CONH₂, -COR⁵, -CH=CRCO₂R⁵, -OCOR⁵, phényle, phényle substitué avec W, -C=CCO₂R⁵, -CH=CHR⁵ ou -C≡CR⁵ ;
chaque groupe R⁵ représente, indépendamment, un groupe alkyle en C₁ à C₄ droit ou ramifié, phényle ou benzyle, ledit groupe phényle et le groupement phényle dudit groupe benzyle pouvant être facultativement substitués avec des substituants halogéno, hydroxy, nitro, cyano, amino, alkyle en C₁ à C₄ droit ou ramifié, alkoxy en C₁ à C₄ droit ou ramifié, phényle, benzyle, alkylamino en C₁ à C₄, di(alkyle en C₁ à C₄) amino ou -S(O)ₘ-(alkyle en C₁ à C₄) droit ou ramifié ;
chaque groupe W représente, indépendamment, un groupe halogéno, R⁵, hydroxy, -OR⁵, nitro, amino, -NHR⁵, -NR⁵R⁵, cyano ou -S(O)ₘ-R⁵ ;
m est égal à 0, 1 ou 2 ;
n a une valeur de 1 à 7 ;
p est égal à 0 ou 1 ;
E¹ et E² sont choisis, indépendamment, entre un atome d'hydrogène, des groupes halogéno, alkyle en C₁ à C₃, hydroxy, alkoxy en C₁ à C₃, nitro, trifluorométhyle, cyano, amino, alkylamino en C₁ à C₃ et di(alkyle en C₁ à C₃) amino ;
Het' et Het" sont choisis, indépendamment, parmi des noyaux hétérocycliques hexagonaux contenant un à quatre atomes d'azote en tant qu'une partie du noyau, facultativement substitués avec un substituant choisi entre des substituants alkyle en C₁ à C₃, halogéno, hydroxy, alkoxy en C₁ à C₃, amino, alkylamino en C₁ à C₃ et di(alkyle en C₁ à C₃) amino ;
(c) des composés de formule III 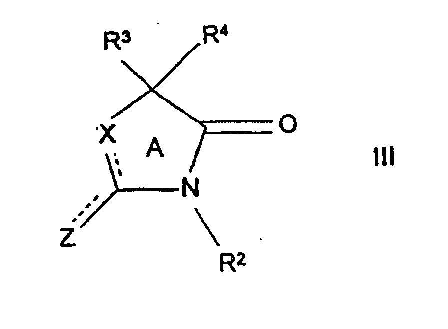 dans laquelle :
les deux lignes discontinues représentent des doubles liaisons facultatives ;
Z représente un atome d'oxygène ou de soufre lorsqu'il est doublement lié au noyau A et Z représente un groupe hydroxy, (alkyle en C₁ à C₁₀)-S-, (alkyle en C₁ à C₁₀)-SO-, (alkyle en C₁ à C₁₀)-SO₂-, adamant-2-yle-S-, naphtyle-S-, benzyle-S-, phényle-C(=O)CH₂-S-, (alkyle en C₁ à C₆)-O-C(=O)-CH₂-S- ou (H, H), (ce qui signifie que Z représente deux atomes d'hydrogène, dont chacun est lié par une liaison simple au même atome de carbone du noyau A) lorsque Z est doublement lié au noyau A, et lesdits groupes naphtyle et phényle et le groupement phényle dudit groupe benzyle peuvent être facultativement substitués avec un à trois substituants choisis, indépendamment, entre des substituants alkyle en C₁ à C₆ facultativement substitués avec un à trois atomes de fluor, alkoxy en C₁ à C₆ facultativement substitués avec un à trois atomes de fluor, halogéno (par exemple chloro, fluoro, bromo ou iodo), amino, alkylamino en C₁ à C₆, di(alkyle en C₁ à C₆)amino, cyano, nitro, (alkyle en C₁ à C₆)-SOₙ- dans lequel n est égal à zéro, un ou deux, -COOH, -COO(alkyle en C₁ à C₆) et -C(O)NH(alkyle en C₁ à C₆) ;
X représente un groupe NR¹ ou CHR¹ ;
R¹ représente un atome d'hydrogène, un groupe alkyle en C₁ à C₆ ou (alkyle en C₁ à C₆)phényle lorsque le noyau A est saturé (c'est-à-dire lorsque le noyau A ne contient aucune double liaison) et R¹ est absent lorsque le noyau A contient une double liaison ;
R² est choisi entre les groupes naphtyle, phényle, (alkyle en C₁ à C₆)phényle, 1-adamantyle, 2-adamantyle, alkyle en C₁ à C₈ droit ou ramifié, cycloalkyle en C₃ à C₁₀ et alkyle en C₈ à C₃₀ bicyclique ou tricyclique ; ledit groupe cycloalkyle en C₃ à C₁₀ et ledit groupe alkyle en C₈ à C₃₀ bicyclique ou tricyclique pouvant être facultativement substitués avec un groupe hydroxy ; et lesdits groupes adamantyle pouvant être facultativement substitués avec un à trois substituants choisis, indépendamment, entre des substituants alkyle en C₁ à C₆, halogéno et hydroxy ; et
R³ et R⁴ sont choisis, indépendamment, parmi des groupes benzyle, le groupement phényle dudit groupe benzyle pouvant être facultativement substitué avec un groupe amino ou nitro ; hydrogène, phényle, (N≡C)-(alkyle en C₁ à C₆), (alkyle en C₁ à C₆)-O-C(=O)-(alkyle en C₁ à C₆) et Het-CH₂, dans lequel Het est choisi entre des groupes 2-, 3- ou 4-pyridinyle, furyle, tétrahydrofuryle, pyrimidyle, pyrazinyle, pyrazolyle, isoxazolyle, thiophényle et triazolyle ;
sous réserve que (a) pas plus d'une des deux lignes discontinues ne puisse représenter une double liaison dans n'importe quel composé, (b) lorsque Z représente un groupe (H, H), X représente un groupe CH₂, (c) lorsque Z représente un atome d'oxygène ou un groupe (H, H) et X représente un groupe CHR¹, R¹ représente l'hydrogène, (d) lorsque Z représente un atome de soufre et X représente un groupe NR¹, R¹ représente un atome d'hydrogène, et (e) un des groupes R³ et R⁴ représente un groupe Het-CH₂ ; et
(d) le composé IV 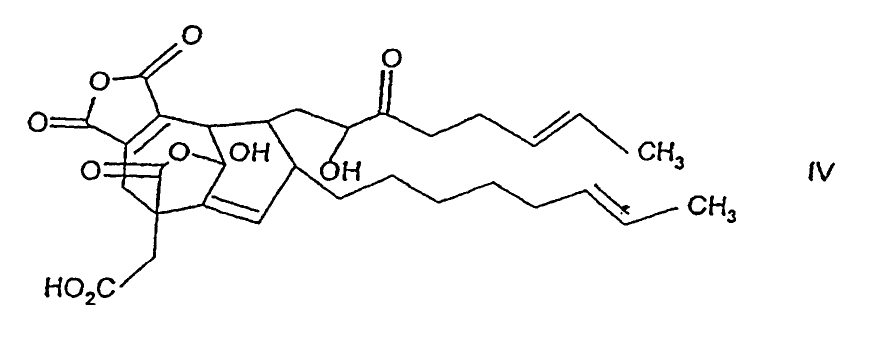 et les sels pharmaceutiquement acceptables des composés précités.

2. Composition pharmaceutique suivant la revendication 1, dans laquelle l'inhibiteur de HMG-CoA-réductase est choisi dans le groupe consistant en l'atorvastatine, la pravastatine, la lovastatine, la compactine, la fluvastatine et la simvastatine, et les sels pharmaceutiquement acceptables des composés précités.

3. Composition suivant la revendication 1 ou 2, destinée à être utilisée comme médicament.

4. Utilisation d'un inhibiteur de FTase répondant à la définition suivant la revendication 1 et d'un inhibiteur de HMG-CoA-réductase dans la préparation d'un médicament destiné au traitement du cancer ou d'un trouble prolifératif bénin chez un mammifère.

5. Utilisation suivant la revendication 4, dans laquelle l'inhibiteur de HMG-CoA-réductase répond à la définition suivant la revendication 2.

6. Composition pharmaceutique pour inhiber la croissance anormale de cellules chez un mammifère, comprenant un inhibiteur de FTase, un inhibiteur de HMG-CoA-réductase et un support pharmaceutiquement acceptable, dans laquelle l'inhibiteur de FTase et l'inhibiteur de HMG-CoA-réductase sont présents en des quantités qui rendent la composition efficace dans l'inhibiteur de la croissance de cellules anormales et dans laquelle l'inhibiteur de FTase est choisi entre :
(a) des composés de formule I 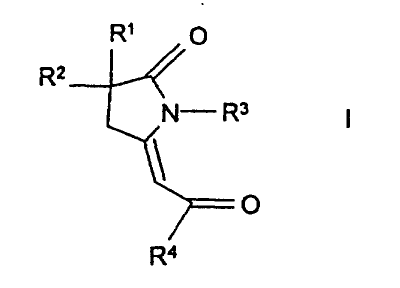 dans laquelle :
R¹ et R² sont choisis, indépendamment, dans le groupe consistant en des groupes-(CH₂)ₚ(hétérocycles penta- à décagonaux), -(CH₂)ₚ(aryle en C₆ à C₁₀), allyle, propargyle et alkyle en C₁ à C₆ dans lesquels p a une valeur de 0 à 3, lesdits groupes alkyle et les groupements alkyle desdits groupes R¹ et R² sont facultativement substitués avec 1 à 3 substituants R⁹, et les groupements aryle et hétérocyclique desdits groupes R¹ et R² sont facultativement substitués avec 1 à 3 substituants choisis, indépendamment, entre des substituants halogéno et R⁹ ;
R³ représente un groupe -(CH₂)ₘ(1- ou 2-adamantyle), -(CH₂)ₘ(cycloalkyle en C₃ à C₁₀), -(CH₂)ₘ(aryle en C₆ à C₁₀), alkyle en C₁ à C₁₀, 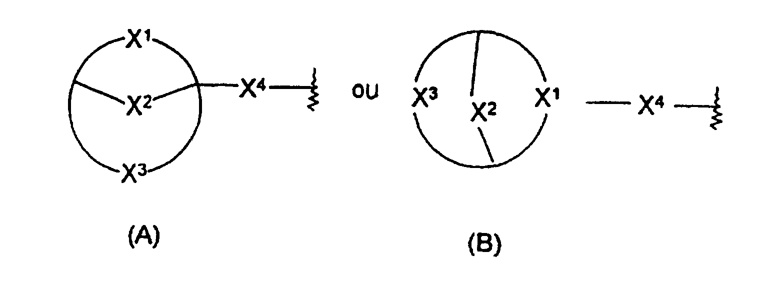 dans lequel m a une valeur de 0 à 6, et lesdits groupes cycloalkyle et alkyle contiennent facultativement 1
ou 2 doubles ou triples liaisons ;
X¹, X² et X³ représentent chacun, indépendamment, un groupe alkylène en C₁ à C₇ contenant facultativement 1 ou 2 doubles ou triples liaisons, X⁴ représente une liaison ou un groupe alkylène en C₁ à C₇ contenant facultativement 1 ou 2 doubles ou triples liaisons et, dans la formule (B), le groupement X⁴ est fixé au groupement X¹ au niveau de n'importe quel atome de carbone disponible dans le groupement X¹ ;
R⁴ représente un groupe aryle en C₆ à C₁₀, hétérocycle penta- à décagonal ou alkyle en C₁ à C₆, chacun desdits groupes R⁴ étant facultativement substitué avec 1 à 3 substituants R⁵ ;
chaque groupe R⁵ est choisi, indépendamment, dans le groupe consistant en des groupes halogéno, nitro, cyano, phényle, -C(O)OR⁶, -SO₂NR⁶R⁷, -NR⁶R⁸, -C(O)R⁶, -OR⁶, -C(O)NR⁶R⁸, -OC(O)NR⁶R⁸, -NR⁸C(O)NR⁸R⁶, -NR⁸C(O)R⁶, -NR⁸C(O)O(alkyle en C₁ à C₄), -C(NR⁸)NR⁸R⁶, -C (NCN)NR⁸R⁶, -C(NCN)S(alkyle en C₁ à C₄), -NR⁸C(NCN)S(alkyle en C₁ à C₄), -NR⁸C(NCN)NR⁸R⁶, -NR⁸SO₂(alkyle en C₁ à C₄), -S(O)ₙ(alkyle en C₁ en C₄) dans lequel n a une valeur de 0 à 2, -NR⁸C(O)C(O)NR⁸R⁶, -NR⁸C(O)C(O)R⁸, thiazolyle, imidazolyle, oxazolyle, pyrazolyle, triazolyle, tétrazolyle et alkyle en C₁ à C₄ facultativement substitué avec 1 à 3 substituants fluoro ;
chacun des groupes R⁶ et R⁷ représente, indépendamment, un atome d'hydrogène ou un groupe alkyle en C₁ à C₄ ;
chaque groupe R⁸ représente, indépendamment, un groupe R⁶ ou -OR⁶ ; et
chaque groupe R⁹ est choisi, indépendamment, entre des groupes cyano, R⁶, -OR⁶, -OC(O)R⁶, -C(O)OR⁶, -C(O)NR⁶R⁷, -NR⁶R⁷, -NR⁶R⁸, -SO₂NR⁶R⁷ et alkyle en C₁ à C₄ substitué avec un substituant hydroxy ;
(b) des composés de formule IIA ou IIB 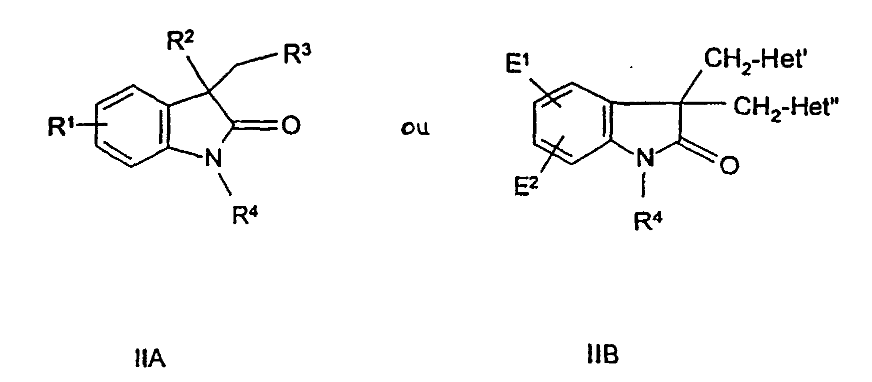 dans laquelle :
R¹ représente un atome d'hydrogène, un groupe halogéno (par exemple chloro, fluoro, bromo ou iodo), cyano, hydroxy, nitro, trifluorométhyle, -NHR⁵, -NR⁵R⁵, R⁵, -OR⁵ ou -S(O)ₘ-R⁵ ;
R² représente un groupe -(CH₂)ₙ-Y ou -OCOR⁵ ;
R³ représente un groupe 4-, 3- ou 2-pyridyle, pyrimidyle, pyrazinyle, 2-fluoro-4-pyridyle ou 3-fluoro-4-pyridyle ;
R⁴ représente un groupe 1-adamantyle ou 2-adamantyle ;
Y représente un atome d'hydrogène, un groupe hydroxy, amino, cyano, -NHR⁵, -NR⁵R⁵, -NHCOR⁵, -NHCO₂R⁵, halogéno, OR⁵, -S(O)ₘR⁵, -CO₂H, -CO₂R⁵, -CONR⁵R⁵, -CONHR⁵, -CONH₂, -COR⁵, -CH=CHCO₂R⁵, -OCOR⁵, phényle, phényle substitué avec W, -C=CCO₂R⁵, -CH=CHR⁵ ou -C≡CR⁵ ;
chaque groupe R⁵ représente, indépendamment, un groupe alkyle en C₁ à C₄ droit ou ramifié, phényle ou benzyle, ledit groupe phényle et le groupement phényle dudit groupe benzyle pouvant être facultativement substitués avec des substituants halogéno, hydroxy, nitro, cyano, amino, alkyle en C₁ à C₄ droit ou ramifié, alkoxy en C₁ à C₄ droit ou ramifié, phényle, benzyle, alkylamino en C₁ à C₄, di(alkyle en C₁ à C₄) amino ou -S(O)ₘ-(alkyle en C₁ à C₄) droit ou ramifié ;
chaque groupe W représente, indépendamment, un groupe halogéno, R⁵, hydroxy, -OR⁵, nitro, amino, -NHR⁵, -NR⁵R⁵, cyano ou -S(O)ₘ-R⁵ ;
m est égal à 0, 1 ou 2 ;
n a une valeur de 1 à 7 ;
p est égal à 0 ou 1 ;
E¹ et E² sont choisis, indépendamment, entre un atome d'hydrogène, des groupes halogéno, alkyle en C₁ à C₃, hydroxy, alkoxy en C₁ à C₃, nitro, trifluorométhyle, cyano, amino, alkylamino en C₁ à C₃ et di(alkyle en C₁ à C₃)amino ;
Het' et Het" sont choisis, indépendamment, parmi des noyaux hétérocycliques hexagonaux contenant un à quatre atomes d'azote en tant qu'une partie du noyau, facultativement substitués avec un substituant choisi entre des substituants alkyle en C₁ à C₃, halogéno, hydroxy, alkoxy en C₁ à C₃, amino, alkylamino en C₁ à C₃ et di(alkyle en C₁ à C₃)amino ;
(c) des composés de formule III 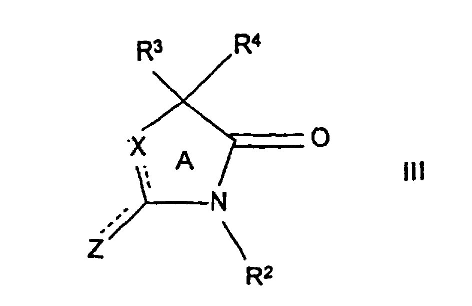 dans laquelle :
les deux lignes discontinues représentent des doubles liaisons facultatives ;
Z représente un atome d'oxygène ou de soufre lorsqu'il est doublement lié au noyau A et Z représente un groupe hydroxy, (alkyle en C₁ à C₁₀)-S-, (alkyle en C₁ à C₁₀)-SO-, (alkyle en C₁ à C₁₀)-SO₂-, adamant-2-yle-S-, naphtyle-S-, benzyle-S-, phényle-C(=O)CH₂-S-, (alkyle en C₁ à C₆)-O-C(=O)-CH₂-S- ou (H,H), (ce qui signifie que Z représente deux atomes d'hydrogène, dont chacun est lié par une liaison simple au même atome de carbone du noyau A) lorsque Z est lié par une liaison simple au noyau A, et lesdits groupes naphtyle et phényle et le groupement naphtyle dudit groupe benzyle peuvent être facultativement substitués avec un à trois substituants choisis, indépendamment, entre des substituants alkyle en C₁ à C₆ facultativement substitués avec un à trois atomes de fluor, alkoxy en C₁ à C₆ facultativement substitués avec un à trois atomes de fluor, halogéno (par exemple chloro, fluoro, bromo ou iodo), amino, alkylamino en C₁ à C₆, di(alkyle en C₁ à C₆)amino, cyano, nitro, (alkyle en C₁ à C₆)-SOₙ- dans lequel n est égal à zéro, un ou deux, -COOH, -COO(alkyle en C₁ à C₆) et -C(O)NH(alkyle en C₁ à C₆) ;
X représente un groupe NR¹ ou CHR¹ ;
R¹ représente un atome d'hydrogène, un groupe alkyle en C₁ à C₆ ou (alkyle en C₁ à C₆)phényle lorsque le noyau A est saturé (c'est-à-dire lorsque le noyau A ne contient aucune double liaison) et R¹ est absent lorsque le noyau A contient une double liaison ;
R² est choisi entre les groupes naphtyle, phényle, (alkyle en C₁ à C₆)phényle, 1-adamantyle, 2-adamantyle, alkyle en C₁ à C₈ droit ou ramifié, cycloalkyle en C₃ à C₁₀ et alkyle en C₈ à C₃₀ bicyclique ou tricyclique ; ledit groupe cycloalkyle en C₃ à C₁₀ et ledit groupe alkyle en C₈ à C₃₀ bicyclique ou tricyclique pouvant être facultativement substitués avec un groupe hydroxy ; et lesdits groupes adamantyle pouvant être facultativement substitués avec un à trois substituants choisis, indépendamment, entre des substituants alkyle en C₁ à C₆, halogéno et hydroxy ; et
R³ et R⁴ sont choisis, indépendamment, parmi des groupes benzyle, le groupement phényle dudit groupe benzyle pouvant être facultativement substitué avec un groupe amino ou nitro ; hydrogène, phényle, (N≡C)-(alkyle en C₁ à C₆), (alkyle en C₁ à C₆)-O-C(=O)-(alkyle en C₁ à C₆) et Het-CH₂, dans lequel Het est choisi entre des groupes 2-, 3- ou 4-pyridinyle, furyle, tétrahydrofuryle, pyrimidyle, pyrazinyle, pyrazolyle, isoxazolyle, thiophényle et triazolyle ;
sous réserve que (a) pas plus d'une des deux lignes discontinues ne puisse représenter une double liaison dans n'importe quel composé, (b) lorsque Z représente un groupe (H, H), X représente un groupe CH₂, (c) lorsque Z représente un atome d'oxygène ou un groupe (H, H) et X représente un groupe CHR¹, R¹ représente un atome d'hydrogène, (d) lorsque Z représente un atome de soufre et X représente un groupe NR¹, R¹ représente un atome d'hydrogène et (e) un des groupes R³ et R⁴ représente un groupe Het-CH₂ ; et
(d) le composé IV 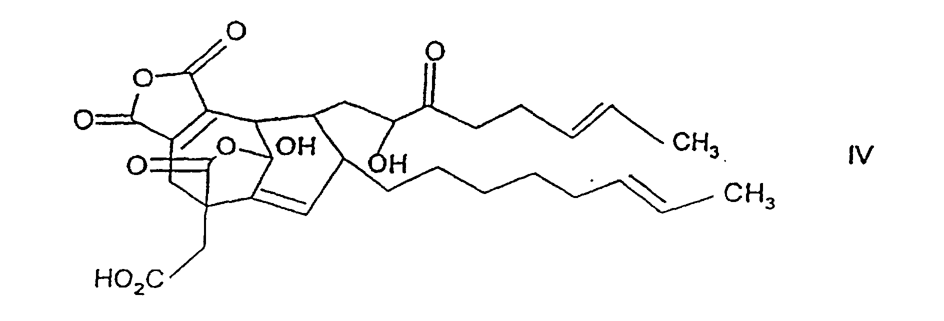 et les sels pharmaceutiquement acceptables des composés précités.

7. Composition pharmaceutique suivant la revendication 5, dans laquelle l'inhibiteur de HMG-CoA-réductase est choisi dans le groupe consistant en l'atorvastatine, la pravastatine, la lovastatine, la compactine, la fluvastatine et la simvastatine, et les sels pharmaceutiquement acceptables des composés précités.

8. Composition suivant la revendication 5 ou 6, destinée à être utilisée comme médicament.

9. Utilisation d'un inhibiteur de FTase répondant à la définition suivant la revendication 5 et d'un inhibiteur de HMG-CoA-réductase dans la préparation d'un médicament destiné au traitement d'une affection chez un mammifère pour lequel l'inhibition de la croissance anormale de cellules est indiquée.

10. Utilisation suivant la revendication 9, dans laquelle l'inhibiteur de HMG-CoA-réductase répond à la définition suivant la revendication 7.
